# EUROPEAN PATENT APPLICATION

(11) **EP 3 434 283 A1**
(43) Date of publication of application: **30.01.2019**
(21) Application number: 17770389.9
(22) Date of filing: 24.03.2017
(51) Int. Cl.: A61K 39/395, A61K 9/08, A61K 9/19, A61K 47/10, A61K 47/12, A61K 47/18, A61K 47/34, A61K 47/60, A61P 19/02, A61P 25/04, A61P 29/00

(54) **MEDICINAL COMPOSITION COMPRISING PEG ANTI-HUMAN NGF ANTIBODY FAB' FRAGMENT**

(30) Priority: 25.03.2016 JP 2016061353
(71) Applicant: Astellas Pharma Inc., Chuo-ku Tokyo 103-8411 (JP)
(72) Inventor: YAMAMOTO Ayano, Tokyo 103-8411 (JP); CHIKUSHI Akinori, Tokyo 103-8411 (JP)
(74) Representative: Hoffmann Eitle
(86) International application number: PCT/JP2017/011910
(87) International publication number: WO 2017/164349

(57) **Abstract**

Provided is a stable pharmaceutical composition comprising a PEGylated Fab' fragment of an anti-human NGF antibody, capable of inhibiting the formation of degradation products, multimers, acidic related substances, basic related substances, and insoluble foreign substances. The pharmaceutical composition has a pH of 4 to 5.5, and can further comprise a pharmaceutically acceptable buffer, a pharmaceutically acceptable isotonizing agent, and a pharmaceutically acceptable surfactant, if desired.

SEQUENCE LISTING

<110>  Astellas Pharma Inc.

<120>  Pharmaceutical composition containing PEGylated Fab' fragment of
       anti-human NGF antibody

<130>  A17003-004

<150>  JP 2016-061353
<151>  2016-03-25

<160>  4

<170>  PatentIn version 3.5

<210>  1
<211>  232
<212>  PRT
<213>  Artificial Sequence

<220>
<223>  H-chain fragment of anti-human NGF antibody Fab' fragment

<400>  1

Gln Val Gln Leu Gln Gln Trp Gly Ala Gly Leu Leu Lys Pro Ser Glu
1               5                   10                  15

Thr Leu Ser Leu Thr Cys Ala Val Tyr Gly Gly Ser Phe Ser Gly Tyr
            20                  25                  30

Tyr Trp Ser Trp Val Arg Gln Pro Pro Gly Lys Gly Leu Glu Trp Ile
        35                  40                  45

Gly Glu Ile Asp His Ser Gly Ser Thr Asn Asn Asn Pro Ser Leu Lys
    50                  55                  60

Ser Arg Val Thr Ile Ser Val Gly Thr Ser Lys Asn Gln Phe Ser Leu
65                  70                  75                  80

Lys Leu Ser Ser Val Thr Ala Ala Asp Thr Ala Val Tyr Tyr Cys Ser
                85                  90                  95

Arg Asp Gly Gly Pro Glu Ser Gly Met Gly Ala Phe Asp Ile Trp Gly
            100                 105                 110

Gln Gly Thr Met Val Thr Val Ser Ser Ala Ser Thr Lys Gly Pro Ser
        115                 120                 125

Val Phe Pro Leu Ala Pro Ser Ser Lys Ser Thr Ser Gly Gly Thr Ala
    130                 135                 140

Ala Leu Gly Cys Leu Val Lys Asp Tyr Phe Pro Glu Pro Val Thr Val

145                 150                 155                 160

Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
            180                 185                 190

Pro Ser Ser Ser Leu Gly Thr Gln Thr Tyr Ile Cys Asn Val Asn His
        195                 200                 205

Lys Pro Ser Asn Thr Lys Val Asp Lys Lys Val Glu Pro Lys Ser Cys
    210                 215                 220

Asp Lys Thr His Thr Cys Ala Ala
225                 230

<210>  2
<211>  699
<212>  DNA
<213>  Artificial Sequence

<220>
<223>  H-chain fragment gene of anti-human NGF antibody Fab' fragment

<400>  2
caggtgcagc tacagcagtg gggcgcagga ctgttgaagc cttcggagac cctgtccctc      60

acctgcgctg tctatggtgg gtccttcagt ggttactact ggagctgggt ccgccagccc     120

ccagggaagg ggctggagtg gattggggaa atcgaccata gtggaagcac caacaacaac     180

ccgtccctca agagtcgagt caccatatca gtaggcacgt ccaagaacca gttctccctg     240

aagctgagct ctgtgaccgc cgcggacacg gctgtgtatt actgttcgag agatgggggc     300

cccgaatcgg ggatgggggc ttttgatatc tggggccaag ggacaatggt caccgtctcc     360

tcagcctcca ccaagggccc atcggtcttc cccctggcac cctcctccaa gagcacctct     420

gggggcacag cggccctggg ctgcctggtc aaggactact tccccgaacc ggtgacggtg     480

tcgtggaact caggcgccct gaccagcggc gtgcacacct tcccggctgt cctacagtcc     540

tcaggactct actccctcag tagcgtggtg accgtgccct ccagcagctt gggcacccag     600

acctacatct gcaacgtgaa tcacaagccc agcaacacca aggtggacaa gaaagttgag     660

cccaaatctt gtgacaaaac tcacacatgc gcagcctga                            699

<210>  3
<211>  219
<212>  PRT
<213>  Artificial Sequence

<220>

<223>  L-chain of anti-human NGF antibody Fab' fragment

<400>  3

Asp Ile Val Met Thr Gln Ser Pro Leu Ser Leu Pro Val Thr Pro Gly
1               5                   10                  15

Glu Pro Ala Ser Ile Ser Cys Arg Ser Ser Gln Ser Leu Leu His Ser
            20                  25                  30

Asn Gly Phe Asn Tyr Leu Gly Trp Tyr Leu Gln Lys Pro Gly Gln Ser
        35                  40                  45

Pro Gln Leu Leu Ile Tyr Leu Gly Ser Asn Arg Ala Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Met Gln Ala
                85                  90                  95

Leu Gln Thr Pro Tyr Thr Phe Gly Gln Gly Thr Lys Leu Glu Ile Lys
            100                 105                 110

Arg Thr Val Ala Ala Pro Ser Val Phe Ile Phe Pro Pro Ser Asp Glu
        115                 120                 125

Gln Leu Lys Ser Gly Thr Ala Ser Val Val Cys Leu Leu Asn Asn Phe
    130                 135                 140

Tyr Pro Arg Glu Ala Lys Val Gln Trp Lys Val Asp Asn Ala Leu Gln
145                 150                 155                 160

Ser Gly Asn Ser Gln Glu Ser Val Thr Glu Gln Asp Ser Lys Asp Ser
                165                 170                 175

Thr Tyr Ser Leu Ser Ser Thr Leu Thr Leu Ser Lys Ala Asp Tyr Glu
            180                 185                 190

Lys His Lys Val Tyr Ala Cys Glu Val Thr His Gln Gly Leu Ser Ser
        195                 200                 205

Pro Val Thr Lys Ser Phe Asn Arg Gly Glu Cys
    210                 215

<210>  4
<211>  660
<212>  DNA

<213>  Artificial Sequence

<220>
<223>  L-chain gene of anti-human NGF antibody Fab' fragment

<400>  4
gatattgtga tgactcagtc tccactctcc ctgcccgtca cccctggaga gccggcctcc      60

atctcctgca ggtctagtca gagcctcctg catagtaatg gattcaacta tttgggttgg     120

tacctgcaga agccagggca gtctccacag ctcctgatct atttgggttc taatcgggcc     180

tccggggtcc ctgacaggtt cagtggcagt ggatcaggca cagattttac tctgaaaatc     240

agcagagtgg aggctgagga tgttggggtt tattactgca tgcaagctct acaaactccg     300

tacacttttg gccaggggac caagctggag atcaaacgga ctgtggctgc accatctgtc     360

ttcatcttcc cgccatctga tgagcagttg aaatctggaa ctgcctctgt tgtgtgcctg     420

ctgaataact tctatcccag agaggccaaa gtacagtgga aggtggataa cgccctccaa     480

tcgggtaact cccaggagag tgtcacagag caggacagca aggacagcac ctacagcctc     540

agcagcaccc tgacgctgag caaagcagac tacgagaaac acaaagtcta cgcctgcgaa     600

gtcacccatc agggcctgag ctcgcccgtc acaaagagct tcaacagggg agagtgttag     660

## Description

### TECHNICAL FIELD

The present invention relates to a stable pharmaceutical composition comprising an anti-human NGF antibody Fab' fragment conjugated with polyethylene glycol.

### TECHNICAL FIELD

A nerve growth factor (NGF) is one of the humoral factors collectively called neurotrophic factors, and plays an important role in the generation, differentiation, and function maintenance of neurons in vivo.

It has been published by the applicant that an anti-human NGF antibody Fab' fragment conjugated with polyethylene glycol (hereinafter polyethylene glycol is referred to as PEG, and "conjugated with PEG" is simply referred to as "PEGylatated") is useful for prevention or treatment of various diseases in which human NGF is involved in disease pathology (for example, osteoarthritis pain (OA pain), rheumatic pain, cancer pain, neuropathic pain, chronic low back pain, postoperative pain, postherpetic neuralgia, painful diabetic neuropathy, fracture pain, painful bladder syndrome, interstitial cystitis, acute pancreatitis, chronic pancreatitis, endometriosis, and the like)(Patent literature 1).

In contrast, various medicines comprising proteins such as antibodies or the like have been developed in recent years, and are actually provided to the medical field. Many of these medicines are administered by intravenous administration, subcutaneous administration, or the like, and thus are provided as a form of a parenteral pharmaceutical composition, such as a liquid preparation, a lyophilized preparation, or the like, to the medical field. In particular, since parenteral pharmaceutical compositions are presumed to be directly administered into the body, stable pharmaceutical preparations are desirable.

That is to say, it is desirable that the formation of foreign insoluble matters and the like is inhibited in a solution containing a protein such as an antibody, and it is desirable that the formation of degradation products, multimers, related substances, and the like is inhibited from the viewpoint of effectiveness.

Therefore, there is still room for improvement in developing a stable pharmaceutical composition comprising the PEGylated anti-human NGF antibody Fab 'fragment.

Patent literatures 2, 3, and 4 disclose inventions relating to a stable pharmaceutical composition containing an anti-human NGF antibody or human NGF, but it is not an invention relating to a PEGylated anti-human NGF antibody Fab' fragment.

### CITATION LIST

### PATENT LITERATURE

[Patent literature 1] WO 2013/022083
[Patent literature 2] WO 2010/032220
[Patent literature 3] WO 95/05845
[Patent literature 4] WO 2011/116090

### SUMMARY OF INVENTION

### TECHNICAL PROBLEM

An object of the present invention is to provide a stable pharmaceutical composition comprising the PEGylated anti-human NGF antibody Fab' fragment.

More particularly, an object of the present invention is to provide a stable pharmaceutical composition comprising the PEGylated anti-human NGF antibody Fab' fragment, capable of inhibiting the generation of degradation products, multimers, acidic related substance, and basic related substances, which increase with, for example, heat, temperature conditions, or the like. Another object of the present invention is to provide a stable pharmaceutical composition comprising the PEGylated anti-human NGF antibody Fab' fragment, capable of inhibiting the generation of foreign insoluble matters, which increase with, for example, freeze-thaw, physical vibration, or the like.

### SOLUTION TO PROBLEM

The present inventor found that a stable pharmaceutical composition could be prepared by adjusting the pH of a solution to a specific range, and formulating the PEGylated anti-human NGF antibody Fab' fragment (Example 1 described below), and that a further stable pharmaceutical composition could be provided by adding specific isotonic agents (Example 2 described below), by adding a surfactant (Example 3 described below), or the like, and completed the present invention.

The present invention relates to:
[1] a stable pharmaceutical composition comprising a PEGylated anti-human NGF antibody Fab' fragment,
   wherein pH is 4 to 5.5;
   wherein the anti-human NGF antibody Fab' fragment is selected from:
   (1) an anti-human NGF antibody Fab' fragment comprising a heavy chain fragment consisting of the amino acid sequence of SEQ ID NO: 1, and a light chain consisting of the amino acid sequence of SEQ ID NO: 3, and
   (2) an anti-human NGF antibody Fab' fragment that is a Fab' fragment generated by a post-translational modification of the anti-human NGF antibody Fab' fragment of (1); and wherein the PEGylation is a covalent bond of a thiol reactive group of a PEG derivative, to which the thiol reactive group is bonded, to a thiol group of cysteine in a hinge region of the anti-human NGF antibody Fab' fragment,
[2] the pharmaceutical composition of [1], further comprising a pharmaceutically acceptable buffer, a pharmaceutically acceptable isotonic agent, and a pharmaceutically acceptable surfactant,
[3] The pharmaceutical composition of[1] or [2], wherein pH is 4.5 to 5.5,
[4] the pharmaceutical composition of any one of[1] to [3], wherein the pharmaceutically acceptable buffer is one, or two or more selected from the group consisting of citric acid, acetic acid, and histidine, and pharmaceutically acceptable salts thereof,
[5] the pharmaceutical composition of any one of[1] to [4], wherein the pharmaceutically acceptable buffer is citric acid, or a pharmaceutically acceptable salt thereof,
[6] the pharmaceutical composition of any one of[1] to [5], wherein a concentration of the pharmaceutically acceptable buffer is 1 to 200 mmol/L,
[7] the pharmaceutical composition of any one of[1] to [6], wherein the pharmaceutical composition is a liquid preparation or a lyophilized preparation,
[8] the pharmaceutical composition of any one of[1] to [7], wherein the pharmaceutical composition is a liquid preparation,
[9] the pharmaceutical composition of any one of[1] to [8], wherein the pharmaceutically acceptable isotonic agent is one, or two or more selected from the group consisting of arginine, D-sorbitol, D-mannitol, trehalose, sucrose, xylitol, erythritol, threitol, inositol, dulcitol, arabitol, isomalt, lactitol, and maltitol,
[10] the pharmaceutical composition of any one of [1] to [9], wherein a concentration of the pharmaceutically acceptable isotonic agent is 1 to 500 mmol/L,
[11] the pharmaceutical composition of any one of [1] to [10], wherein the pharmaceutically acceptable surfactant is at least one of polysorbate or poloxamer,
[12] the pharmaceutical composition of any one of [1] to [11], wherein the pharmaceutically acceptable surfactant is polysorbate 80,
[13] the pharmaceutical composition of any one of [1] to [12], wherein a concentration of the pharmaceutically acceptable surfactant is 0.001 to 1% (w/v),
[14] the pharmaceutical composition of any one of [1] to [13], wherein a concentration of the PEGylated anti-human NGF antibody Fab' fragment is 0.1 to 200 mg/mL,
[15] the pharmaceutical composition of [1], comprising, as the PEGylated anti-human NGF antibody Fab' fragment, an anti-human NGF antibody Fab' fragment PEGylated by covalently bonding a thiol reactive group of a PEG derivative, to which the thiol reactive group is bonded, to a thiol group of cysteine in a hinge region of an anti-human NGF antibody Fab' fragment comprising a heavy chain fragment consisting of the amino acid sequence of SEQ ID NO: 1, and a light chain consisting of the amino acid sequence of SEQ ID NO: 3,
[16] the pharmaceutical composition of [1], comprising, as the PEGylated anti-human NGF antibody Fab' fragment, an anti-human NGF antibody Fab' fragment PEGylated by covalently bonding a thiol reactive group of a PEG derivative, to which the thiol reactive group is bonded, to a thiol group of cysteine in a hinge region of an anti-human NGF antibody Fab' fragment that is a Fab' fragment generated by a post-translational modification of an anti-human NGF antibody Fab' fragment comprising a heavy chain fragment consisting of the amino acid sequence of SEQ ID NO: 1, and a light chain consisting of the amino acid sequence of SEQ ID NO: 3,
[17] the pharmaceutical composition of [1] or [16], wherein the post-translational modification of an anti-human NGF antibody Fab' fragment is pyroglutamylation at the N-terminus of a heavy chain variable region,
[18] a method of stabilizing a PEGylated anti-human NGF antibody Fab' fragment, characterizing by adjusting a pH to 4 to 5.5,
   wherein the anti-human NGF antibody Fab' fragment is selected from:
   (1) an anti-human NGF antibody Fab' fragment comprising a heavy chain fragment consisting of the amino acid sequence of SEQ ID NO: 1, and a light chain consisting of the amino acid sequence of SEQ ID NO: 3, and
   (2) an anti-human NGF antibody Fab' fragment that is a Fab' fragment generated by a post-translational modification of the anti-human NGF antibody Fab' fragment of (1); and wherein the PEGylation is a covalent bond of a thiol reactive group of a PEG derivative, to which the thiol reactive group is bonded, to a thiol group of cysteine in a hinge region of the anti-human NGF antibody Fab' fragment, and
[19] the method of [18], using a pharmaceutically acceptable buffer, a pharmaceutically acceptable isotonic agent, and a pharmaceutically acceptable surfactant.

### ADVANTAGEOUS EFFECTS OF INVENTION

According to the present invention, a stable pharmaceutical composition comprising the PEGylated anti-human NGF antibody Fab' fragment, more particularly, a stable pharmaceutical composition comprising the PEGylated anti-human NGF antibody Fab' fragment, capable of inhibiting the generation of its degradation products, multimers, acidic related substances, and basic related substances, can be provided.

### BRIEF DESCRIPTION OF THE DRAWINGS

Fig. 1 is a graph showing the increased amount (%) of degradation products in the formulations (samples No. D-1 to No. D-7) comprising an isotonic agent (D-mannitol) and a surfactant (polysorbate 80) in the range of pH 4.0 to 6.0, under storage conditions at 25°C or 40°C for 2 weeks. ● shows the results of samples containing 20 mmol/L citric acid as a buffer, and ■ shows the results of samples containing 20 mmol/L histidine as a buffer.
Fig. 2 is a graph showing the increased amount (%) of acidic related substances in the formulations (samples No. D-1 to No. D-7) comprising an isotonic agent (D-mannitol) and a surfactant (polysorbate 80) in the range of pH 4.0 to 6.0, under storage conditions at 25°C or 40°C for 2 weeks. ● shows the results of samples containing 20 mmol/L citric acid as a buffer, and ■ shows the results of samples containing 20 mmol/L histidine as a buffer.
Fig. 3 is a graph showing the amount of multimers (%) in the formulations comprising an isotonic agent (D-mannitol or D-sorbitol) and a surfactant (polysorbate 80) and adjusted to a desired pH (4.6, 4.9, or 5.2) using citric acid, after storage at 5°C, 25°C, or 40°C.
Fig. 4 is a graph showing the amount of degradation products (%) in the formulations comprising an isotonic agent (D-mannitol or D-sorbitol) and a surfactant (polysorbate 80) and adjusted to a desired pH (4.6, 4.9, or 5.2) using citric acid, after storage at 5°C, 25°C, or 40°C.
Fig. 5 is a graph showing the amount of acidic related substances (%) in the formulations comprising an isotonic agent (D-mannitol or D-sorbitol) and a surfactant (polysorbate 80) and adjusted to a desired pH (4.6, 4.9, or 5.2) using citric acid, after storage at 5°C, 25°C, or 40°C.

### DESCRIPTION OF EMBODIMENTS

The term "stable" as used herein means to have stability against, for example, heat, light, temperature, humidity, shaking, and/or freeze-thaw. For example, after a pharmaceutical composition is allowed to store under predetermined conditions, it means that the amount of multimers, degradation products, acidic related substances, or basic related substances, or the total amount thereof, contained in the pharmaceutical composition is a specific amount or less. As another embodiment, it means that no foreign insoluble matter is visually observed after storage of a pharmaceutical composition under predetermined conditions.

The term "multimer" as used herein means a complex produced by collecting PEGylated anti-human NGF antibody Fab' fragments. A measuring method of the multimers is not particularly limited, but includes, for example, size exclusion chromatography (SE-HPLC method), gel electrophoresis, capillary electrophoresis, micro flow imaging, and the like. An embodiment is the SE-HPLC method. In the SE-HPLC method, the amount is calculated as percentage (%), by measuring the area of detected peaks by an automatic analysis method and dividing it by the sum of all peak areas including the main peak. The main peak as used herein means the peak of the active body. The peaks having a retention time shorter than that of the main peak in the SE-HPLC are collectively defined as the multimers. The amount of the multimers measured by the SE-HPLC method is 10% or less as an embodiment, and 5% or less as another embodiment.

The term "degradation product" as used herein is a fragment generated by elimination of part of a PEGylated anti-human NGF antibody Fab' fragment. A measuring method of the degradation products is not particularly limited, but includes, for example, the SE-HPLC method, gel electrophoresis, capillary electrophoresis, and the like. An embodiment is the SE-HPLC method. The measuring method of the degradation products by the SE-HPLC method is carried out in a similar manner to the measurement of the multimers by the SE-HPLC method, as described above. The peaks having a retention time longer than that of the main peak in the SE-HPLC are collectively defined as the degradation products. The amount of the degradation products measured by the SE-HPLC method is 10% or less as an embodiment, and 5% or less as another embodiment.

The increased amount of the degradation products (a difference between the amount of the degradation products at the beginning of a test (storage) and the amount of the degradation products after storage of a predetermined period of time under specific conditions) is, for example, 2.5% or less, and 2.0% or less as an embodiment. As an embodiment, the increased amount of the degradation products after storage at 25°C for 2 weeks or for 1 month (4 weeks), or at 40°C for 2 weeks is, for example, 2.5% or less, and 2.0% or less as an embodiment; as another embodiment, the increased amount of the degradation products after storage at 40°C for 2 weeks is 2.5% or less; and as still another embodiment, the increased amount of the degradation products after storage at 40°C for 2 weeks is 2.0% or less.

The term "acidic related substance" as used herein means a related substance recognized as a peak eluting faster than the main component by cation exchange chromatography or imaging capillary isoelectric focusing. A measuring method of the acidic related substances is not particularly limited, but includes, for example, cation exchange chromatography (IE-HPLC method), gel electrophoresis, capillary electrophoresis, imaging capillary isoelectric focusing (icIEF method), and the like. An embodiment is the IE-HPLC method or the icIEF method. In the IE-HPLC method and the icIEF method, the percentage (%) of peaks is calculated in a similar manner to the SE-HPLC method, as described above. The amount of the acidic related substances measured by the IE-HPLC method and/or the icIEF method is 50% or less as an embodiment, 35% or less as another embodiment, and 25% or less as still another embodiment.

The increased amount of the acidic related substances (a difference between the amount of the acidic related substances at the beginning of a test (storage) and the amount of the acidic related substances after storage of a predetermined period of time under specific conditions) is, for example, 15% or less, and 10% or less as an embodiment. More particularly, the increased amount of the acidic related substances after storage at 25°C for 2 weeks or for 1 month (4 weeks), or at 40°C for 2 weeks is, for example, 15% or less, and 10% or less as an embodiment.

The term "basic related substance" as used herein means a related substance recognized as a peak eluting slower than the main component by the IE-HPLC method or the icIEF method. A measuring method of the basic related substances is not particularly limited, but includes, for example, the IE-HPLC method, gel electrophoresis, capillary electrophoresis, the icIEF method, and the like. An embodiment is the IE-HPLC method or the icIEF method. In the IE-HPLC method and the icIEF method, the percentage (%) of peaks is calculated in a similar manner to the SE-HPLC method, as described above. The amount of the basic related substances measured by the icIEF method is 50% or less as an embodiment, 25% or less as another embodiment, and 20% or less as still another embodiment.

The term "foreign insoluble matter" as used herein means an insoluble complex produced by collecting PEGylated anti-human NGF antibody Fab' fragments, pharmaceutical additives, and the like. The foreign insoluble matters can be visually confirmed, for example, by a Foreign Insoluble Matter Test for Injections described in the Japanese Pharmacopoeia, Sixteenth Edition, or a similar method.

The term "stable pharmaceutical composition" as used herein means a pharmaceutical composition in which, at refrigerating temperature (2 to 8°C) for at least 1 month (4 weeks), preferably 3 months, more preferably 6 months, still more preferably 1 year, or still more preferably 2 years; at room temperature (22 to 28°C) for at least 2 weeks, preferably 1 month, more preferably 3 months, still more preferably 6 months, or still more preferably 1 year; or under accelerated conditions (37 to 43°C) for at least 2 weeks, or preferably for 1 month (4 weeks), the amount of multimers, degradation products, acidic related substances, or basic related substances, or the total amount thereof, is a specific amount or less, as described above.

There are five classes of antibodies: IgG, IgM, IgA, IgD, and IgE. The basic structure of an antibody molecule is common to each class and is composed of a heavy chain having a molecular weight of 50,000 to 70,000 and a light chain of 20,000 to 30,000. The heavy chain usually consists of a polypeptide containing about 440 amino acids, and has a characteristic structure for each class, and the heavy chains of IgG, IgM, IgA, IgD, and IgE are called Igγ, Igµ, Igα, Igδ, and Igε, respectively. IgG further has subclasses: IgG1, IgG2, IgG3, and IgG4, and the heavy chains thereof are called Igγ1, Igγ2, Igγ3, and Igγ4, respectively. The light chain usually consists of a polypeptide containing about 220 amino acids, and two types of light chains, L type and K type, are known, and called Igλ and Igκ, respectively. With respect to the polypeptide constitution of the basic structure of an antibody molecule, two homologous heavy chains and two homologous light chains are linked by disulfide bonds (S-S bond) and noncovalent bonds, and the molecular weight is 150,000 to 190,000. Two types of light chains can pair with any heavy chain. An individual antibody molecule always consists of two identical light chains and two identical heavy chains.

There are four (five for the µ or ε chain) intrachain S-S bonds and two intrachain S-S bonds, and one loop is formed every 100 to 110 amino acid residues, and this steric structure is similar between each loop and is called a structural unit or a domain. The domain located at the amino terminus (N-terminus) for both heavy and light chains is not constant in its amino acid sequence, even if the antibody is a specimen that is the same class (subclass) from the same animal species, and the domain is called a variable region, and each domain is called a heavy chain variable region (V_{H}) and a light chain variable region (V_{L}). The amino acid sequence at the carboxyl terminal (C-terminal) side from the variable region is almost constant for each class or subclass, and is called a constant region, and each domain is represented as C_{H}1, C_{H}2, C_{H}3, or C_{L}, respectively.

The antigenic determinant site of an antibody is constituted with the heavy chain variable region and the light chain variable region, and the binding specificity depends on the amino acid sequence of this site. On the other hand, biological activities such as binding to complements or various cells reflect the differences in the constant region structure among the various classes of Ig. It is known that the variability in the variable regions of the heavy chain and light chain is mostly limited to three small hypervariable regions present in both chains, and these regions are called complementarity determining regions (CDRs; CDR1, CDR2 and CDR3 starting from the N-terminal side). The remaining portion of the variable region is called a framework region (FR) and is relatively constant.

A region between the C_{H}1 domain and the C_{H}2 domain of the heavy-chain constant region of an antibody is called a hinge region. This region includes lots of proline residues and has a plurality of inter-chain S-S bonds connecting two heavy-chains. For example, each hinge region of human IgG1, IgG2, IgG3, and IgG4 includes 2, 4, 11, and 2 cysteine residues respectively which constitute the inter-heavy-chain S-S bonds. The hinge region is a region highly sensitive to a proteolytic enzyme such as papain or pepsin. When an antibody is digested with papain, its heavy chain is cleaved at a position closer to the N-terminal side than to the inter-heavy-chain S-S bond of the hinge region, whereby the antibody is broken down into two Fab fragments and one Fc fragment. The Fab fragment is constituted with a light-chain and a heavy-chain fragment including a heavy-chain variable region (V_{H}), a C_{H}1 domain, and a portion of the hinge region. When an antibody is digested with pepsin, its heavy-chain is cleaved at a position closer to the C-terminal side than to the inter-heavy-chain S-S bond of the hinge region, whereby F(ab')₂ fragments is generated. The F(ab')₂ fragment is a fragment having a dimeric structure in which two Fab' fragments bind to each other via the inter-heavy-chain S-S bond in the hinge region. The Fab' fragment is constituted with a light-chain and a heavy-chain fragment including a heavy-chain variable region (V_{H}), a C_{H}1 domain, and a portion of the hinge region. Cysteine residues constituting the inter-heavy-chain S-S bond are included in the portion of the hinge region. All of the Fab fragment, F(ab')₂ fragment, and Fab' fragment include the variable region and have antigen-binding activity.

It is known that when an antibody is expressed in cells, the antibody undergoes a post-translational modification. As examples of the post-translational modification, cleavage of lysine at the C-terminus of a heavy chain by carboxypeptidase, modification to pyroglutamic acid by pyroglutamylation of glutamine or glutamic acid at the N-terminus of heavy and light chains, or the like, may be exemplified, and it is known that lysine at the C-terminus of a heavy chain is deleted, and modification to pyroglutamic acid occurs for most of the glutamine at the N-terminus of a heavy chain, in various antibodies (Journal of Pharmaceutical Sciences, 2008, Vol. 97, p. 2426). Further, it is known in the art that such a post-translational modification does not affect the antibody activity (Analytical Biochemistry, 2006, Vol. 348, p. 24-39).

The pharmaceutical composition of the present invention comprises, as a PEGylated anti-human NGF antibody Fab' fragment, a PEGylated anti-human NGF antibody Fab' fragment of the following (1) and/or a PEGylated anti-human NGF antibody Fab' fragment of the following (2):
(1) an anti-human NGF antibody Fab' fragment PEGylated by covalently bonding a thiol reactive group of a PEG derivative, to which the thiol reactive group is bonded, to a thiol group of cysteine in a hinge region of an anti-human NGF antibody Fab' fragment comprising a heavy chain variable region consisting of the amino acid sequence of amino acids 1 to 121 of SEQ ID NO: 1, and a light chain variable region consisting of the amino acid sequence of amino acids 1 to 113 of SEQ ID NO: 3,
(2) an anti-human NGF antibody Fab' fragment PEGylated by covalently bonding a thiol reactive group of a PEG derivative, to which the thiol reactive group is bonded, to a thiol group of cysteine in a hinge region of an anti-human NGF antibody Fab' fragment that is a Fab' fragment generated by a post-translational modification of the anti-human NGF antibody Fab' fragment of (1).
(WO 2013/022083)

In an embodiment, the post-translational modification of the anti-human NGF antibody Fab' fragment of (2) is pyroglutamylation at the N-terminus of the heavy chain valuable region. In an embodiment, the anti-human NGF antibody Fab' fragment of (2) is an anti-human NGF antibody Fab' fragment PEGylated by covalently bonding a thiol reactive group of a PEG derivative, to which the thiol reactive group is bonded, to a thiol group of cysteine in a hinge region of an anti-human NGF antibody Fab' fragment comprising a heavy chain variable region consisting of the amino acid sequence of amino acids 1 to 121 of SEQ ID NO: 1 in which glutamine of amino acid number 1 of SEQ ID NO: 1 is modified to pyroglutamic acid, and a light chain variable region consisting of the amino acid sequence of amino acids 1 to 113 of SEQ ID NO: 3.

As the constant regions of the PEGylated anti-human NGF antibody Fab' fragment used in the present invention, any subclass of constant region (for example, a constant region of Igγ1, Igγ2, Igγ3, or Igγ4 as the heavy chain constant region, and a constant region of Igλ or Igκ as the light chain constant region) may be selectable. Preferably, the PEGylated anti-human NGF antibody Fab' fragment used in the present invention contains the heavy chain constant region of human Igγ1 constant region, as the heavy chain constant region. Preferably, the PEGylated anti-human NGF antibody Fab' fragment used in the present invention contains the light chain constant region of human Igκ constant region, as the light chain constant region. Preferably, the PEGylated anti-human NGF antibody Fab' fragment used in the present invention contains the heavy chain constant region of human Igγ1 constant region and the light chain constant region of human Igκ constant region.

In an embodiment, the pharmaceutical composition of the present invention comprises, as the PEGylated anti-human NGF antibody Fab' fragment, a PEGylated anti-human NGF antibody Fab' fragment of the following (3) and/or a PEGylated anti-human NGF antibody Fab' fragment of the following (4):
(3) an anti-human NGF antibody Fab' fragment PEGylated by covalently bonding a thiol reactive group of a PEG derivative, to which the thiol reactive group is bonded, to a thiol group of cysteine in a hinge region of an anti-human NGF antibody Fab' fragment comprising a heavy chain fragment consisting of the amino acid sequence of SEQ ID NO: 1, and a light chain consisting of the amino acid sequence of SEQ ID NO: 3,
(4) an anti-human NGF antibody Fab' fragment PEGylated by covalently bonding a thiol reactive group of a PEG derivative, to which the thiol reactive group is bonded, to a thiol group of cysteine in a hinge region of an anti-human NGF antibody Fab' fragment that is a Fab' fragment generated by a post-translational modification of an anti-human NGF antibody Fab' fragment comprising a heavy chain fragment consisting of the amino acid sequence of SEQ ID NO: 1, and a light chain consisting of the amino acid sequence of SEQ ID NO: 3.

In an embodiment, the post-translational modification of the anti-human NGF antibody Fab' fragment of (4) is pyroglutamylation at the N-terminus of the heavy chain valuable region. In an embodiment, the anti-human NGF antibody Fab' fragment of (4) is an anti-human NGF antibody Fab' fragment PEGylated by covalently bonding a thiol reactive group of a PEG derivative, to which the thiol reactive group is bonded, to a thiol group of cysteine in a hinge region of an anti-human NGF antibody Fab' fragment comprising a heavy chain variable region consisting of the amino acid sequence of SEQ ID NO: 1 in which glutamine of amino acid number 1 of SEQ ID NO: 1 is modified to pyroglutamic acid, and a light chain variable region consisting of the amino acid sequence of SEQ ID NO: 3.

In an embodiment, the pharmaceutical composition of the present invention comprises, as the PEGylated anti-human NGF antibody Fab' fragment, a PEGylated anti-human NGF antibody Fab' fragment of the following (1) and a PEGylated anti-human NGF antibody Fab' fragment of the following (2):
(1) an anti-human NGF antibody Fab' fragment PEGylated by covalently bonding a thiol reactive group of a PEG derivative, to which the thiol reactive group is bonded, to a thiol group of cysteine in a hinge region of an anti-human NGF antibody Fab' fragment comprising a heavy chain variable region consisting of the amino acid sequence of amino acids 1 to 121 of SEQ ID NO: 1, and a light chain variable region consisting of the amino acid sequence of amino acids 1 to 113 of SEQ ID NO: 3,
(2) an anti-human NGF antibody Fab' fragment PEGylated by covalently bonding a thiol reactive group of a PEG derivative, to which the thiol reactive group is bonded, to a thiol group of cysteine in a hinge region of an anti-human NGF antibody Fab' fragment that is a Fab' fragment generated by a post-translational modification of the anti-human NGF antibody Fab' fragment of (1).

In an embodiment, the pharmaceutical composition of the present invention comprises, as the PEGylated anti-human NGF antibody Fab' fragment, a PEGylated anti-human NGF antibody Fab' fragment of the following (3) and/or a PEGylated anti-human NGF antibody Fab' fragment of the following (4):
(3) an anti-human NGF antibody Fab' fragment PEGylated by covalently bonding a thiol reactive group of a PEG derivative, to which the thiol reactive group is bonded, to a thiol group of cysteine in a hinge region of an anti-human NGF antibody Fab' fragment comprising a heavy chain fragment consisting of the amino acid sequence of SEQ ID NO: 1, and a light chain consisting of the amino acid sequence of SEQ ID NO: 3,
(4) an anti-human NGF antibody Fab' fragment PEGylated by covalently bonding a thiol reactive group of a PEG derivative, to which the thiol reactive group is bonded, to a thiol group of cysteine in a hinge region of an anti-human NGF antibody Fab' fragment that is a Fab' fragment generated by a post-translational modification of an anti-human NGF antibody Fab' fragment comprising a heavy chain fragment consisting of the amino acid sequence of SEQ ID NO: 1, and a light chain consisting of the amino acid sequence of SEQ ID NO: 3.

The present invention also includes a pharmaceutical composition comprising an anti-human NGF antibody Fab' fragment PEGylated by covalently bonding a thiol reactive group of a PEG derivative, to which the thiol reactive group is bonded, to a thiol group of cysteine in a hinge region of an anti-human NGF antibody Fab' fragment having the following characteristics, as the PEGylated anti-human NGF antibody Fab' fragment: an anti-human NGF antibody Fab' fragment comprising a heavy chain valuable region comprising CDR1 consisting of the amino acid sequence of amino acids 31 to 35 of SEQ ID NO: 1, CDR2 consisting of the amino acid sequence of amino acids 50 to 65 of SEQ ID NO: 1, and CDR3 consisting of the amino acid sequence of amino acids 98 to 110 of SEQ ID NO: 1, and a light chain valuable region comprising CDR1 consisting of the amino acid sequence of amino acids 24 to 39 of SEQ ID NO: 3, CDR2 consisting of the amino acid sequence of amino acids 55 to 61 of SEQ ID NO: 3, and CDR3 consisting of the amino acid sequence of amino acids 94 to 102 of SEQ ID NO: 3.

The present invention also includes a pharmaceutical composition comprising an anti-human NGF antibody Fab' fragment PEGylated by covalently bonding a thiol reactive group of a PEG derivative, to which the thiol reactive group is bonded, to a thiol group of cysteine in a hinge region of an anti-human NGF antibody Fab' fragment obtainable by culturing a host cell selected from the following (a) and (b), as the PEGylated anti-human NGF antibody Fab' fragment:
(a) a host cell transformed with an expression vector comprising a polynucleotide comprising a nucleotide sequence encoding a heavy chain fragment comprising the heavy chain variable region consisting of the amino acid sequence of amino acids 1 to 121 of SEQ ID NO: 1, and a polynucleotide comprising a nucleotide sequence encoding a light chain comprising the light chain variable region consisting of the amino acid sequence of amino acids 1 to 113 of SEQ ID NO: 3,
(b) a host cell transformed with an expression vector comprising a polynucleotide comprising a nucleotide sequence encoding a heavy chain fragment comprising the heavy chain variable region consisting of the amino acid sequence of amino acids 1 to 121 of SEQ ID NO: 1, and an expression vector comprising a polynucleotide comprising a nucleotide sequence encoding a light chain comprising the light chain variable region consisting of the amino acid sequence of amino acids 1 to 113 of SEQ ID NO: 3.

The present invention also includes a pharmaceutical composition comprising an anti-human NGF antibody Fab' fragment PEGylated by covalently bonding a thiol reactive group of a PEG derivative, to which the thiol reactive group is bonded, to a thiol group of cysteine in a hinge region of an anti-human NGF antibody Fab' fragment obtainable by culturing a host cell selected from the following (a) and (b), as the PEGylated anti-human NGF antibody Fab' fragment:
(a) a host cell transformed with an expression vector comprising a polynucleotide comprising a nucleotide sequence encoding the heavy chain fragment consisting of the amino acid sequence of SEQ ID NO: 1, and a polynucleotide comprising a nucleotide sequence encoding the light chain consisting of the amino acid sequence of SEQ ID NO: 3,
(b) a host cell transformed with an expression vector comprising a polynucleotide comprising a nucleotide sequence encoding the heavy chain fragment consisting of the amino acid sequence of
SEQ ID NO: 1, and an expression vector comprising a polynucleotide comprising a nucleotide sequence encoding the light chain consisting of the amino acid sequence of SEQ ID NO: 3.

In the present invention, the Fab' fragment is a monovalent antibody fragment consisting of a light chain, and a heavy chain fragment comprising a heavy chain variable region (V_{H}), C_{H}1 domain, and part of the hinge region. This part of the hinge region contains at least one cysteine residue (also called "hinge region cysteine") other than the cysteine residue constituting the S-S bond between the heavy chain and the light chain. The hinge region cysteine can be used as a modification site with PEG. The number of the hinge region cysteines in the Fab' fragment may vary between one and several depending on the class of antibody used, but is easily adjustable by those skilled in the art. For example, when the Fab' fragment of the human IgG1 class (generally, having two hinge region cysteines in the hinge region) is prepared, a Fab' fragment having one hinge region cysteine in the hinge region may be prepared by inserting a stop codon between the coding site of the first hinge region cysteine and the coding site of the second hinge region cysteine, and a Fab' fragment having two hinge region cysteines in the hinge region may be prepared by inserting a stop codon after the coding site of the second hinge region cysteine.

The PEGylated anti-human NGF antibody Fab' fragment used in the present invention can be prepared by performing PEGylation by covalently bonding a thiol reactive group of a PEG derivative, to which the thiol reactive group is bonded, to a thiol group of cysteine in a hinge region of an anti-human NGF antibody Fab' fragment. The conjugation of PEG to the Fab' fragment may be carried out using a known method in the art (for example, European Patent No. 0948544). In the present invention, any linear or branched PEG having any average molecular weight, or its derivative may be used, and may be easily selected by those skilled in the art depending on the intended use. To facilitate the conjugation of PEG to the hinge region cysteine, a derivative of PEG may be used. For example, a PEG derivative in which a thiol reactive group such as maleimide is bonded via a linker is used to covalently bond the maleimide group to a thiol group of the hinge region cysteine. The average molecular weight is generally approximately 500 to approximately 50,000 Da, preferably approximately 5,000 to approximately 40,000 Da, and more preferably approximately 10,000 to approximately 40,000 Da.

The PEGylated anti-human NGF antibody Fab' fragment used in the present invention can be easily prepared by those skilled in the art, using a known method in the art, based on the sequence information of the anti-human NGF antibody Fab' fragment disclosed herein. As a method of preparing the PEGylated anti-human NGF antibody Fab' fragment used in the present invention, a method disclosed in WO 2013/022083 may be exemplified.

The amount of the PEGylated anti-human NGF antibody Fab' fragment in one unit pharmaceutical composition (preparation) is, for example, 0.1 to 200 mg, 0.1 to 40 mg as an embodiment, 20 to 40 mg as another embodiment, 60 to 150 mg as still another embodiment, and 40 to 120 mg as still another embodiment, in terms of Fab'-PEG. Each lower limit and each upper limit can be arbitrarily combined as desired.

When the pharmaceutical composition is in a solid state (for example, a lyophilized preparation, a spray-dried preparation, or the like), the amount of the PEGylated anti-human NGF antibody Fab' fragment is, for example, 0.1 to 200 mg, 0.1 to 40 mg as an embodiment, 20 to 40 mg as another embodiment, 60 to 150 mg as still another embodiment, and 40 to 120 mg as still another embodiment, in terms of Fab'-PEG. Each lower limit and each upper limit can be arbitrarily combined as desired.

The concentration of the PEGylated anti-human NGF antibody Fab' fragment when dissolved at the time of use is, for example, 0.1 to 200 mg/mL, 0.1 to 40 mg/mL as an embodiment, 20 to 40 mg/mL as another embodiment, 60 to 150 mg/mL as still another embodiment, and 40 to 120 mg/mL as still another embodiment, in terms of Fab'-PEG after dissolved at the time of use. Each lower limit and each upper limit can be arbitrarily combined as desired.

When the pharmaceutical composition is a liquid state (solution), the concentration of the PEGylated anti-human NGF antibody Fab' fragment is, for example, 0.1 to 200 mg/mL, 0.1 to 40 mg/mL as an embodiment, 20 to 40 mg/mL as another embodiment, 60 to 150 mg/mL as still another embodiment, and 40 to 120 mg/mL as still another embodiment, in terms of Fab'-PEG. Each lower limit and each upper limit can be arbitrarily combined as desired.

The dose is, for example, 0.1 to 200 mg, 0.1 to 40 mg as an embodiment, 20 to 40 mg as another embodiment, 60 to 150 mg as still another embodiment, and 40 to 120 mg as still another embodiment, in terms ofFab'-PEG. Each lower limit and each upper limit can be arbitrarily combined as desired.

Indications include prevention and/or treatment of various diseases in which human NGF is involved in disease pathology, for example, osteoarthritis pain (OA pain), rheumatic pain, cancer pain, neuropathic pain, chronic low back pain, postoperative pain, postherpetic neuralgia, painful diabetic neuropathy, fracture pain, painful bladder syndrome, interstitial cystitis, acute pancreatitis, chronic pancreatitis, endometriosis, and the like.

A "pharmaceutically acceptable buffer" used in the present invention is not particularly limited, so long as it is pharmaceutically acceptable, and in a solution state, the pH of the solution can be adjusted within the desired pH range.

More particularly, it has buffering capacity within the following pH range. The pH is, for example, 4 to 5.5, 4.0 to 5.5 in an embodiment, 4.5 to 5.5 in another embodiment, and 4.6 to 5.2 in still another embodiment. Each lower limit and each upper limit can be arbitrarily combined as desired.

When the pharmaceutical composition is a liquid preparation, the pH is defined as the pH of the liquid preparation, and when the pharmaceutical composition is a lyophilized preparation or a spray-dried preparation, the pH is defined as the pH of a solution obtained by dissolving the preparation in water.

As the buffer component, for example, citric acid, acetic acid, or histidine, or a pharmaceutically acceptable salt thereof, or the like, may be included as an embodiment. As another embodiment, citric acid, acetic acid, or a pharmaceutically acceptable salt thereof, or the like, may be included. As still another embodiment, anhydrous citric acid and/or trisodium citrate dihydrate may be included.

One kind or two or more kinds of these buffer components can be appropriately used in appropriate amounts. Hydrochloric acid, sodium hydroxide, or the like may be appropriately added in order to adjust the pH within a desired pH range.

The concentration of the buffer is not particularly limited, so long as the pH can be adjusted within the desired pH range. When the pharmaceutical composition is in a solution state (liquid preparation) dissolved with water for injection, the concentration of the buffer is, for example, 1 to 200 mmol/L, 1 to 100 mmol/L in an embodiment, 5 to 100 mmol/L in another embodiment, and 1 to 50 mmol/L in still another embodiment. Each lower limit and each upper limit can be arbitrarily combined as desired.

When the pharmaceutical composition is a lyophilized preparation or a spray-dried preparation, the amount of the buffer in a solution when the preparation is redissolved in water is the above-mentioned concentration.

A "pharmaceutically acceptable isotonic agent" used in the present invention is not particularly limited, so long as it is pharmaceutically acceptable, it has isotonic action, and it is one not affecting stability of the PEGylated anti-human NGF antibody Fab' fragment or having stabilizing action.

More particularly, for example, salts, amino acids, sugars, or sugar alcohols, may be exemplified.

As the salts, for example, sodium chloride, calcium chloride, magnesium chloride, sodium sulfate, calcium sulfate, or the like, may be exemplified. It is sodium chloride in an embodiment.

As the amino acids, for example, arginine (L-arginine) or a pharmaceutically acceptable salt thereof (for example, hydrochloride), glycine, lysine, ornithine, or the like, may be exemplified. It is arginine or glycine in an embodiment, and it is arginine in another embodiment.

As the sugars or sugar alcohols, for example, D-sorbitol, D-mannitol, trehalose, sucrose, xylitol, erythritol, threitol, inositol, dulcitol, arabitol, isomalt, lactitol, maltitol, or the like, may be exemplified. It is D-sorbitol or D-mannitol in an embodiment, and it is D-sorbitol in another embodiment.

As the isotonic agent, it is arginine or a pharmaceutically acceptable salt thereof, D-sorbitol, D-mannitol, trehalose, sucrose, xylitol, erythritol, inositol, arabitol, isomalt, lactitol, or maltitol in an embodiment, and it is L-arginine hydrochloride or D-sorbitol in another embodiment.

One kind or two or more kinds of these isotonic agents can be appropriately used.

Within the scope of not affecting the stability of the PEGylated anti-human antibody Fab' fragment or having stabilizing action, these salts, amino acids, sugars, or sugar alcohols may be included in the pharmaceutical composition.

The concentration of the isotonic agent is not particularly limited, so long as it has isotonic action, and it does not affect the stability of the PEGylated anti-human antibody Fab' fragment.

When the pharmaceutical composition is in a solution state (liquid preparation) dissolved with water for injection, the concentration of the isotonic agent is, for example, 1 to 500 mmol/L, 1 to 400 mmol/L in an embodiment, 1 to 300 mmol/L in another embodiment, 50 to 500 mmol/L in still another embodiment, 100 to 300 mmol/L in still another embodiment, 200 to 300 mmol/L in still another embodiment, and 100 to 200 mmol/L in still another embodiment. Each lower limit and each upper limit can be arbitrarily combined as desired.

When the pharmaceutical composition is a lyophilized preparation or a spray-dried preparation, the amount of the isotonic agent in a solution when the preparation is redissolved in water is the above-mentioned concentration.

A "pharmaceutically acceptable surfactant" used in the present invention is not particularly limited, so long as it is pharmaceutically acceptable, it has surfactant action, and it has stabilizing action of the PEGylated anti-human NGF antibody Fab' fragment.

More particularly, for example, nonionic surfactants (for example, sorbitan fatty acid esters, such as sorbitan monocaprylate, sorbitan monolaurate, sorbitan monopalmitate, and the like; glycerin fatty acid esters, such as glycerol monocaprylate, glycerol monomyristate, glycerol monostearate, and the like; polyglycerol fatty acid esters, such as decaglyceryl monostearate, decaglyceryl distearate, decaglyceryl monolinoleate, and the like; polyoxyethylene sorbitan fatty acid esters, such as polyoxyethylene sorbitan monolaurate, polyoxyethylene sorbitan monooleate, polyoxyethylene sorbitan monostearate, polyoxyethylene sorbitan monopalmitate, polyoxyethylene sorbitan trioleate, polyoxyethylene sorbitan tristearate, and the like; polyoxyethylene sorbitol fatty acid ester, such as polyoxyethylene sorbitol tetrastearate, polyoxyethylene sorbitol tetraoleate, and the like; polyoxyethylene glycerin fatty acid esters, such as polyoxyethylene glyceryl monostearate, and the like; polyethylene glycol fatty acid esters, such as polyethylene glycol distearate, and the like; polyoxyethylene alkyl ethers, such as polyoxyethylene lauryl ether, and the like; polyoxyethylene polyoxypropylene alkyl ethers, such as polyoxyethylene polyoxypropylene glycol ether, polyoxyethylene polyoxypropylene propyl ether, polyoxyethylene polyoxypropylene cetyl ether, and the like; polyoxyethylene alkylphenyl ethers, such as polyoxyethylene nonylphenyl ether, and the like; polyoxyethylene hydrogenated castor oil, such as polyoxyethylene castor oil, polyoxyethylene hydrogenated castor oil, and the like; polyoxyethylene beeswax derivatives, such as polyoxyethylene sorbitol beeswax, and the like; polyoxyethylene lanolin derivatives, such as polyoxyethylene lanolin, and the like; and surfactants having an HLB of 6 to 18, such as polyoxyethylene fatty acid amides, and the like (for example, polyoxyethylene octadecanamide, and the like)); anionic surfactants (for example, alkyl sulfates having a C₁₀-C₁₈ alkyl group, such as sodium cetyl sulfate, sodium lauryl sulfate, sodium oleyl sulfate, and the like; polyoxyethylene alkyl ether sulfate in which the average number of moles of added ethylene oxide units is 2 to 4 and the number of carbon atoms of the alkyl group is 10 to 18, such as sodium polyoxyethylene lauryl sulfate, and the like; alkyl sulfosuccinate having C₈-C₁₈ alkyl group, such as sodium lauryl sulfosuccinate, and the like); natural surfactants, such as lecithin, glycerophospholipid, and the like; sphingophospholipids, such as sphingomyelin, and the like; and sucrose esters of C₁₂-C₁₈ fatty acids, are included.

One kind or two or more kinds of these surfactants can be appropriately selected and used.

The surfactant is polyoxyethylene sorbitan fatty acid esters or polyoxyethylene polyoxypropylene alkyl ethers as an embodiment, polysorbates or pluronic-type surfactants as another embodiment, polysorbates 20, 21, 40, 60, 65, 80, 81, or 85, or pluronic-type surfactants as still another embodiment, polysorbates 20 or 80, or poloxamer (poloxamer 188) as still another embodiment, and polysorbate 80 as still another embodiment.

The surfactant has an action of inhibiting the formation of foreign insoluble matters and the like, and within the scope of stabilizing the PEGylated anti-human antibody Fab' fragment, the surfactant may be included in the pharmaceutical composition.

The concentration of the surfactant is not particularly limited, so long as it has surfactant action and stabilizing action of the PEGylated anti-human NGF antibody Fab' fragment.

When the pharmaceutical composition is in a solution state (liquid preparation) dissolved with water for injection, the concentration of the surfactant is, for example, 0.001 to 1% (w/v), 0.01 to 0.5% (w/v) in an embodiment, and 0.01 to 0.03% (w/v) in another embodiment. Each lower limit and each upper limit can be arbitrarily combined as desired.

When the pharmaceutical composition is a lyophilized preparation or a spray-dried preparation, the amount of the surfactant in a solution when the preparation is redissolved in water is the above-mentioned concentration.

The pharmaceutical composition of the present invention can be provided as a parenteral pharmaceutical composition, such as a liquid preparation by filling a container with the solution, or a lyophilized preparation, a spray-dried preparation, or the like obtained by subjecting the solution to lyophilization or spray-drying. The preferred pharmaceutical composition is a liquid preparation.

Among the pharmaceutical compositions of the present invention, particularly liquid preparations comprising citric acid, arginine, and polysorbate, or liquid preparations comprising citric acid, D-sorbitol, and polysorbate are preferred.

To the pharmaceutical composition of the present invention, pharmaceutical additives, such as a suspending agent, a solubilizing agent, a preserving agent, an adsorption inhibitor, a sulfur-containing reducing agent, an antioxidant agent, or the like, can be appropriately added, if desired.

As the suspending agent, for example, methyl cellulose, hydroxyethyl cellulose, gum arabic, tragacanth powder, sodium carboxymethyl cellulose, polyoxyethylene sorbitan monolaurate, and the like, may be exemplified.

As the solubilization agent, for example, polyoxyethylene hydrogenated castor oil, nicotinamide, polyoxyethylene sorbitan monolaurate, macrogol, castor oil fatty acid ethyl ester, and the like, may be exemplified.

As the preserving agent, for example, methyl parahydroxybenzoate, ethyl parahydroxybenzoate, sorbic acid, phenol, cresol, chlorocresol, and the like, may be exemplified.

As the adsorption inhibitor, for example, human serum albumin, lecithin, dextran, an ethylene oxide/propylene oxide copolymer, hydroxypropyl cellulose, methyl cellulose, polyoxyethylene hydrogenated castor oil, polyethylene glycol, and the like, may be exemplified.

As the sulfur-containing reducing agent, for example, N-acetyl cysteine, N-acetyl homocysteine, thioctic acid, thiodiglycol, thioethanolamine, thioglycerol, thiosorbitol, thioglycolic acid and a salt thereof, sodium thiosulfate, glutathione, a compound having a sulfhydryl group, such as a thioalkanoic acid having 1 to 7 carbon atoms, and the like, may be exemplified.

As the antioxidant agent, for example, methionine, erythorbic acid, dibutylhydroxytoluene, butylhydroxyanisole, α-tocopherol, tocopherol acetate, L-ascorbic acid and a salt thereof, L-ascorbyl palmitate, L-ascorbic acid stearate, sodium bisulfite, sodium sulfite, triamyl gallate, propyl gallate, or chelating agents, such as disodium ethylenediaminetetraacetate (EDTA), sodium pyrophosphate, sodium metaphosphate, and the like, may be exemplified.

These pharmaceutical additives can be appropriately used in an appropriate amount within a range of an amount capable of achieving the desired effect of the present invention.

The method of preparing a pharmaceutical composition of the present invention includes a method of comprising a PEGylated anti-human NGF antibody Fab' fragment and adjusting pH to 4 to 5.5, by a known preparation method per se, and it can further comprise a pharmaceutically acceptable buffer, a pharmaceutically acceptable isotonic agent, and a pharmaceutically acceptable surfactant, if desired.

The container, which is filled with the pharmaceutical composition of the present invention, may be selected in accordance with the purpose of use. The container includes ones having a form of a specified capacity, such as a vial, an ampoule, and a syringe, or ones with a large capacity, such as a bottle. The container includes a syringe (including a disposable syringe) as an embodiment. By filling the syringe with a solution in advance and providing it as a prefilled syringe solution preparation, an operation such as a dissolution operation or the like becomes unnecessary in the medical field, a prompt response is expected.

With respect to the material of the container, glass, plastics, or the like, may be exemplified. With respect to the surface treatment in the container, a silica coating treatment, a silicone coating treatment, a sulfur treatment, a various low alkali treatment, and the like, may be carried out.

The present invention includes a method of stabilizing a PEGylated anti-human NGF antibody Fab' fragment, characterizing by adjusting a pH to 4 to 5.5. In the stabilizing method of the present invention, a pharmaceutically acceptable buffer, a pharmaceutically acceptable isotonic agent, and a pharmaceutically acceptable surfactant can be further used, if desired.

With respect to the terms "PEGylated anti-human NGF antibody Fab' fragment", "pharmaceutically acceptable buffer", "pharmaceutically acceptable isotonic agent", and "pharmaceutically acceptable surfactant" used in the stabilizing method of the present invention, the above-mentioned explanations in the pharmaceutical composition of the present invention can be applied as they are. Further, with respect to the contents of these components, the formulation method thereof, and the like, the above-mentioned explanations in the pharmaceutical composition of the present invention can be applied as they are.

In the stabilizing method of the present invention, the generation of degradation products, multimers, acidic related substances, basic related substances, and foreign insoluble matters can be inhibited by adjusting pH to 4 to 5.5 in preparing the PEGylated anti-human NGF antibody Fab' fragment, and further using a pharmaceutically acceptable buffer, a pharmaceutically acceptable isotonic agent, and a pharmaceutically acceptable surfactant, if desired.

### EXAMPLES

The present invention will now be further illustrated by, but is by no means limited to, the following Referential Example and Examples.

The PEGylated anti-human NGF antibody Fab' fragment used in the Examples was obtained by the method described in Example 19 of WO 2013/022083 or a similar method.

### «Referential Example: Preparation of PEGylated anti-human NGF antibody Fab' fragment»

With respect to the anti-human NGF antibody Fab' fragment used in the Examples, the nucleotide sequence encoding the heavy chain is shown in SEQ ID NO: 2, the amino acid sequence encoded thereby is shown in SEQ ID NO: 1, the nucleotide sequence encoding the light chain is shown in SEQ ID NO: 4, and the amino acid sequence encoded thereby is shown in SEQ ID NO: 3. The CDR1, CDR2, and CDR3 in the heavy chain variable region of the anti-human NGF antibody Fab' fragment consist of the amino acid sequences of amino acids 31 to 35, amino acids 50 to 65, and 98 to 110 of SEQ ID NO: 1, respectively. The CDR1, CDR2, and CDR3 in the light chain variable region of the anti-human NGF antibody Fab' fragment consist of the amino acid sequences of amino acids 24 to 39, amino acids 55 to 61, and 94 to 102 of SEQ ID NO: 3, respectively.

In accordance with WO 2013/022083, a GS vector (Lonza) into which both genes for the heavy chain fragment and the light chain of the anti-human NGF antibody Fab' fragment were inserted was constructed. CHOK1SV cells (Lonza) were transfected with the vector to obtain a stable expression strain of the Fab' fragment, and the Fab' fragment was expressed. The culture supernatant was purified using an affinity chromatography and an anion exchange chromatography to obtain the Fab' fragment. In connection with this, the steps included a virus inactivation step and a virus removal membrane step.

Next, the obtained anti-human NGF antibody Fab' fragment was reduced with a reducing agent, and coupled with PEG maleimide (product name: SUNBRIGHT GL2-400MA, manufactured by NOF CORPORATION). The reaction product was purified using a cation exchange chromatography to prepare a PEGylated anti-human NGF antibody Fab' fragment. As a result of analyzing the amino acid modification of the purified Fab' fragment, almost all of the N-terminus of the heavy chain variable region was pyroglutamylated.

### <<Example 1: Stabilization effect by selection of optimum pH>>

In order to adjust the concentration of the PEGylated anti-human NGF antibody Fab' fragment to 10 mg/mL in terms of Fab' (approximately 20 mg/mL in terms of Fab'-PEG), formulation liquids (samples No. A-1 to No. A-8) prepared by dilution with buffers (each concentration was 20 mmol/L) shown in Table 1 were sterilely filtered through a 0.22 µm filter, and glass vials (3 mL volume) were filled with 1.3 mL of each filtrate, and capping with rubber stoppers and crimping were carried out, and these vials were stored at 25°C for a month in an inverted position.

In connection with this, hydrochloric acid and/or sodium hydroxide were added during buffer preparation as pH adjusters, if necessary, so as to become a desired pH.

The concentration in terms of Fab', and the concentration in terms of Fab'-PEG were measured using an ultraviolet/visible spectrophotometer (Nanodrop 2000c; Thermo Scientific, MA, U.S.A). After 2 µL of each sample was applied to a stage, absorbance at 280 nm was measured. The protein concentration was calculated using the obtained absorbance at 280 nm, and an extinction coefficient of 1.48 mL/mg/cm (in terms of Fab' concentration) or an extinction coefficient of 0.79 mL/mg/cm (in terms of Fab'-PEG concentration).

The evaluation results of an SE-HPLC method and an IE-HPLC method are shown in Table 1.

In the SE-HPLC measurement, a TSKgel (registered trademark) G3000 SWXL column (manufactured by Tosoh) was connected to an HPLC system. A mobile phase prepared by adding acetonitrile, so as to be 10%, to a solution of 10 mmol/L phosphoric acid/500 mmol/L sodium chloride pH 7.0, was flowed at a flow rate of 0.5 mL/min. Each sample was injected in an amount of 50 µg in terms of Fab' (approximately 100 µg in terms of Fab'-PEG). The column temperature was set to 30°C, and the sample temperature was set to 5°C. The detection was carried out at UV 280 nm.

The amounts were calculated as percentage (%) by measuring the area of peaks detected by the SE-HPLC measurement by an automatic analysis method, and dividing it by the sum of all peaks including the main peak. The main peak as used herein means the peak of the active body. The peaks having a retention time shorter than that of the main peak in the SE-HPLC were collectively defined as multimers, and the peaks having a retention time longer than that of the main peak were collectively defined as degradation products.

In the IE-HPLC measurement, a Propac WCX-10 column (manufactured by Dionex) was connected to an HPLC system, and the measurement was carried out. A mobile phase of 20 mmol/L morpholinoethanesulfonic acid (MES)/pH 6.0 was connected to a mobile phase A line, and a mobile phase of 20 mmol/L MES/500 mmol/L sodium chloride/pH 6.0 was connected to a mobile phase B line, and the mobile phases were flowed at a flow rate of 1 mL/min. Each sample was diluted with the mobile phase A to a concentration of 1 mg/mL in terms of Fab', and 10 µL was injected. In the gradient program, after mobile phase B was held at 0% for 2 minutes, the proportion of mobile phase B was linearly increased to 5% in 50 minutes. Subsequently, after linearly increasing the proportion of mobile phase B to 100% and washing, the proportion of mobile phase B was linearly reduced to 0%, and held for approximately 10 minutes, and equilibration was carried out until the next sample injection. The analysis time was approximately 70 minutes, and the detection was carried out at UV 280 nm. The column temperature was set to 40°C, and the sample temperature was set to 5°C.

The percentage of peaks was calculated by a method similar to SE-HPLC.

The peaks around 12 minutes of the IE-HPLC in the test were defined as acidic related substances.

As a result, an increase in multimers, degradation products, and acidic related substances were observed in A-5 around pH 7.

It was shown from the results that the generation of multimers, degradation products, and acidic related substances was inhibited by formulating the PEGylated anti-human NGF antibody Fab' fragment around pH 4-6.

**[Table 1]**

| Sample No. | Buffer | pH | Multimers (%) | | Degradation products (%) | | Acidic related substances (%) | |
|---|---|---|---|---|---|---|---|---|
| | | | Initial | Storage 1 week | Initial | Storage 1 week | Initial | Storage 1 week |
| A-1 | Citric acid | 4 | 1.21 | 1.33 | 4.40 | 5.77 | 42.98 | 40.78 |
| A-2 | | 5 | 1.27 | 1.38 | 4.40 | 4.81 | 43.63 | 45.67 |
| A-3 | | 6 | 1.31 | 1.50 | 4.45 | 4.24 | 43.33 | 62.19 |
| A-4 | Phosphoric acid | 6 | 1.37 | 1.57 | 4.41 | 4.69 | 43.72 | 66.37 |
| A-5 | | 7 | 1.62 | 1.94 | 4.66 | 6.96 | 46.86 | 96.13 |
| A-6 | Acetic acid | 5 | 1.22 | 1.36 | 4.47 | 4.91 | 43.11 | 45.89 |
| A-7 | Succinic acid | 6 | 1.33 | 1.53 | 4.44 | 5.19 | 43.58 | 62.88 |
| A-8 | Histidine | 6 | 1.31 | 1.37 | 4.55 | 5.32 | 44.34 | 65.94 |

### <<Example 2: Stabilization effect by selection of isotonic agents>>

The stability of the PEGylated anti-human NGF antibody Fab' fragment (the amount of multimers, the amount of degradation products, and polydispersity) was evaluated in formulations (samples No. B-1 to No. B-7) prepared by adding six kinds of pharmaceutical additives (L-arginine hydrochloride, sodium chloride, glycine, D-sorbitol, sucrose, and D-mannitol) at a concentration to be isotonic, to a buffer formulation consisting of 20 mg/mL PEGylated anti-human NGF antibody Fab' fragment in terms of Fab' (approximately 40 mg/mL in terms of Fab'-PEG) and 20 mmol/L citric acid (pH5.5). The samples prepared in accordance with each formulation were sterilely filtered through a 0.22 µm filter, and glass vials (3 mL volume) were filled with 0.3 mL of each filtrate, and capping with rubber stoppers and crimping were carried out, and these vials were stored at 40°C for 4 weeks in an upright position. The results are shown in Table 2.

In connection with this, hydrochloric acid and/or sodium hydroxide were added during buffer preparation as pH adjusters, if necessary, so as to become a desired pH.

The amount of multimers and the amount of degradation products were analyzed by the SE-HPLC method in a similar manner to Example 1.

The polydispersity was measured by dynamic light scattering (DLS), using DynaPro Platereader (manufactured by Wyatt).

In the formulation containing sodium chloride, there was a tendency to increase the amount of multimers and the amount of degradation products after the storage at 40°C for 4 weeks, in comparison with the no additive formulation.

In the formulation containing L-arginine hydrochloride and the formulation containing glycine, an increase in the amount of degradation products was observed after the storage at 40°C for 4 weeks, in comparison with the no additive formulation. In the formulation containing D-sorbitol and the formulation containing D-mannitol, the amount of multimers, the amount of degradation products, and the polydispersity after the storage at 40°C for 4 weeks were similar to those of the no additive formulation.

In connection with this, with respect of the formulations in which the amount of degradation products increased, in comparison with the no additive formulation, the stability is comprehensively judged, taking into consideration the results of evaluation for the amounts of multimers, acidic related substances, basic related substances, and the like.

**[Table 2]**

| Sample No. | Isotonic agent | Multimers (%) | | Degradation products (%) | | Polydispersity (%) | |
|---|---|---|---|---|---|---|---|
| | | Initial | Storage 4 weeks | Initial | Storage 4 weeks | Initial | Storage 4 weeks |
| B-1 | Not Added | 1.61 | 1.93 | 4.54 | 6.91 | 19.9 | 23.8 |
| B-2 | 140 mmol/L L-Arginine hydrochloride | 1.61 | 1.61 | 4.58 | 7.47 | 20.6 | 22.3 |
| B-3 | 140 mmol/L Sodium chloride | 1.67 | 1.99 | 4.57 | 7.12 | 15.0 | 19.5 |
| B-4 | 240 mmol/L Glycine | 1.64 | 1.64 | 4.58 | 8.01 | 19.0 | 22.8 |
| B-5 | 240 mmol/L D-Sorbitol | 1.69 | 1.86 | 4.57 | 6.91 | 31.7 | 23.8 |
| B-6 | 240 mmol/L Sucrose | 1.69 | 1.80 | 4.57 | 6.90 | 32.8 | Multimodal |
| B-7 | 240 mmol/L D-mannitol | 1.63 | 1.87 | 4.54 | 6.86 | 21.0 | 23.7 |

| | | | | | | | |
|---|---|---|---|---|---|---|---|
| Multimodal : Beyond measurable range | | | | | | | |

### <<Example 3: Stabilization effect by addition of surfactants>>

The physical stability against freeze-thaw stress was evaluated in samples (samples No. C-1 to No. C-4) prepared by adding three kinds of concentration levels (0.01, 0.02, and 0.05% (w/v)) of polysorbate 80, which was a surfactant, to a formulation consisting of 20 mg/mL PEGylated anti-human NGF antibody Fab' fragment in terms of Fab' (approximately 40 mg/mL in terms of Fab'-PEG) and 20 mmol/L citric acid (pH5.5). The samples prepared in accordance with each formulation were sterilely filtered through a 0.22 µm filter, and glass vials (3 mL volume) were filled with 0.3 mL of each filtrate, and capping with rubber stoppers and crimping were carried out.

A freeze-thaw stress test was carried out as follows. After the samples were stored and frozen in a freezer at -80°C in an upright position, the samples were taken out of the freezer, and stored and thawed in a refrigerator at 5°C in an upright positon. This freeze-thaw cycle was repeated three times.

In connection with this, hydrochloric acid and/or sodium hydroxide were added during buffer preparation as pH adjusters, if necessary, so as to become a desired pH.

The results of appearance evaluation by visual inspection are shown in Table 3.

In the appearance evaluation, generation of a large number of foreign insoluble matters due to freeze-thaw stress was observed in the formulation not containing polysorbate 80, whereas no foreign insoluble matter was observed at any concentration in the formulations containing polysorbate 80.

**[Table 3]**

| Sample No. | Surfactant concentration (% (w/v)) | Initial | After freeze-thaw stress test |
|---|---|---|---|
| C-1 | 0 | No foreign insoluble matter appeared | Foreign insoluble matter appeared |
| C-2 | 0.01 | No foreign insoluble matter appeared | No foreign insoluble matter appeared |
| C-3 | 0.02 | No foreign insoluble matter appeared | No foreign insoluble matter appeared |
| C-4 | 0.05 | No foreign insoluble matter appeared | No foreign insoluble matter appeared |

### <<Example 4: Stabilization effect by selection of optimum pH in formulations containing isotonic agent and surfactant 1>>

With respect to formulations (samples No. D-1 to No. D-7) consisting of 20 mg/mL PEGylated anti-human NGF antibody Fab' fragment in terms of Fab' (approximately 40 mg/mL in terms of Fab'-PEG), 20 mmol/L citric acid or histidine as a buffer, 240 mmol/L D-mannitol, and 0.02% (w/v) polysorbate 80, samples were prepared in the range of pH 4.0 to 6.0, and the stability after storage at 25°C or 40°C in an upright position was evaluated by the SE-HPLC method, the IE-HPLC method, and the DLS method. The samples prepared in accordance with each formulation were sterilely filtered through a 0.22 µm filter, and glass vials (3 mL volume) were filled with 0.3 mL of each filtrate, and capping with rubber stoppers and crimping were carried out.

In connection with this, hydrochloric acid and/or sodium hydroxide were added during buffer preparation as pH adjusters, if necessary, so as to become a desired pH.

The measurements by the SE-HPLC method and the IE-HPLC method were carried out in a similar manner to Example 1, and the measurement by the DLS method was carried out in a similar manner to Example 2.

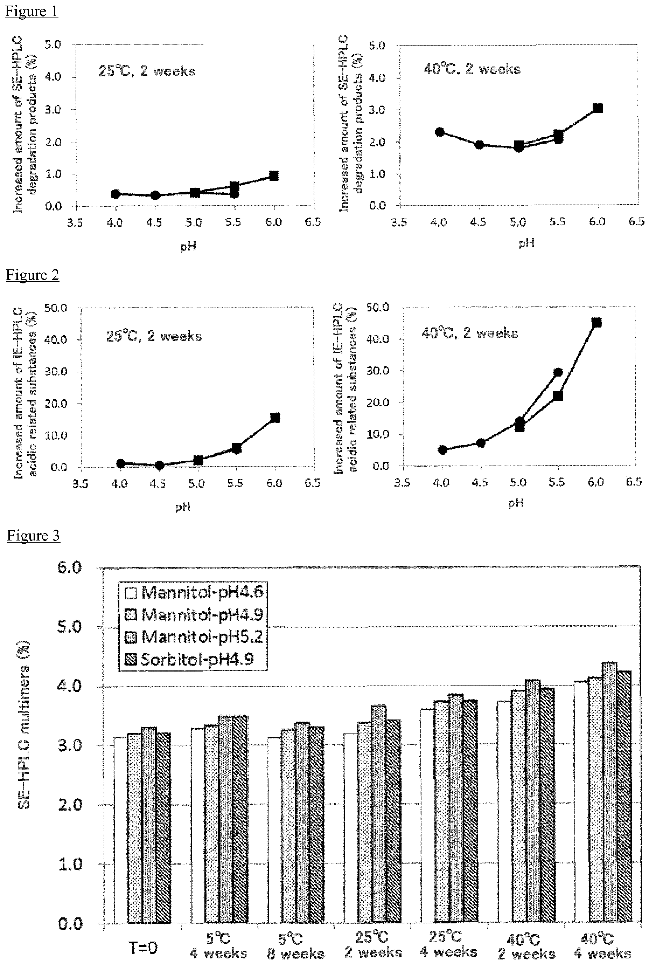

The results are shown in Figure 1, Figure 2, and Table 4.

The degradation products showed the smallest increase at pH 5.0, in comparison with the initial sample. The acidic related substances tended to increase the amount of increase from the initial sample, as the pH was higher, in any of the storage conditions.

From the results of the DLS measurement shown in Table 4, in the storage at 40°C for 2 weeks, the lower the pH, the larger the average radius and the polydispersity value, and the tendency to form aggregates was observed.

**[Table 4]**

| Sample No. | Buffer | pH | Average radius (nm) | | | Polydispersity (%) | | |
|---|---|---|---|---|---|---|---|---|
| | | | Initial | Storage 25°C 2 weeks | Storage 40°C 2 weeks | Initial | Storage 25°C 2 weeks | Storage 40°C 2 weeks |
| D-1 | Citric acid | 4.0 | 4.5 | 4.5 | 5.4 | 23.0 | 22.7 | Multimodal |
| D-2 | | 4.5 | 4.6 | 4.7 | 5.1 | 20.4 | 20.6 | 49.9 |
| D-3 | | 5.0 | 4.8 | 4.8 | 5.0 | 20.2 | 21.0 | 23.8 |
| D-4 | | 5.5 | 4.8 | 4.7 | 5.0 | 20.5 | 22.2 | 23.8 |
| D-5 | Histidine | 5.0 | 4.6 | 4.7 | 4.7 | 21.3 | 21.2 | 23.9 |
| D-6 | | 5.5 | 4.6 | 4.6 | 4.7 | 22.5 | 22.2 | 23.7 |
| D-7 | | 6.0 | 4.8 | 4.8 | 4.7 | 23.8 | 31.1 | 23.3 |

| | | | | | | | | |
|---|---|---|---|---|---|---|---|---|
| Multimodal: Beyond measurable range | | | | | | | | |

### <<Example 5: Stabilization effect by selection of optimal pH in formulations containing isotonic agent and surfactant 2>>

With respect to formulations prepared by adding 240 mmol/L D-mannitol and 0.02% (w/v) polysorbate 80, to formulation liquids at pH 4.6, pH 4.9, and pH 5.2 prepared using citric acid (each concentration was 20 mmol/L), and a formulation (pH 4.9) prepared by adding 240 mmol/L D-sorbitol, instead of D-mannitol, the stability after storage at 5°C, 25°C, and 40°C in an upright position was evaluated. In connection with this, the concentration of the PEGylated anti-human NGF antibody Fab' fragment in terms of Fab' was 20mg/mL (approximately 40 mg/mL in terms of Fab'-PEG). The samples prepared in accordance with each formulation were sterilely filtered through a 0.22 µm filter, and glass vials (3 mL volume) were filled with 0.3 mL of each filtrate, and capping with rubber stoppers and crimping were carried out.

In connection with this, hydrochloric acid and/or sodium hydroxide were added during buffer preparation as pH adjusters, if necessary, so as to become a desired pH.

The multimers and the degradation products were measured by the SE-HPLC method, and the acidic related substances were measured by the IE-HPLC method. The SE-HPLC method and the IE-HPLC method were carried out in a similar manner to Example 1.

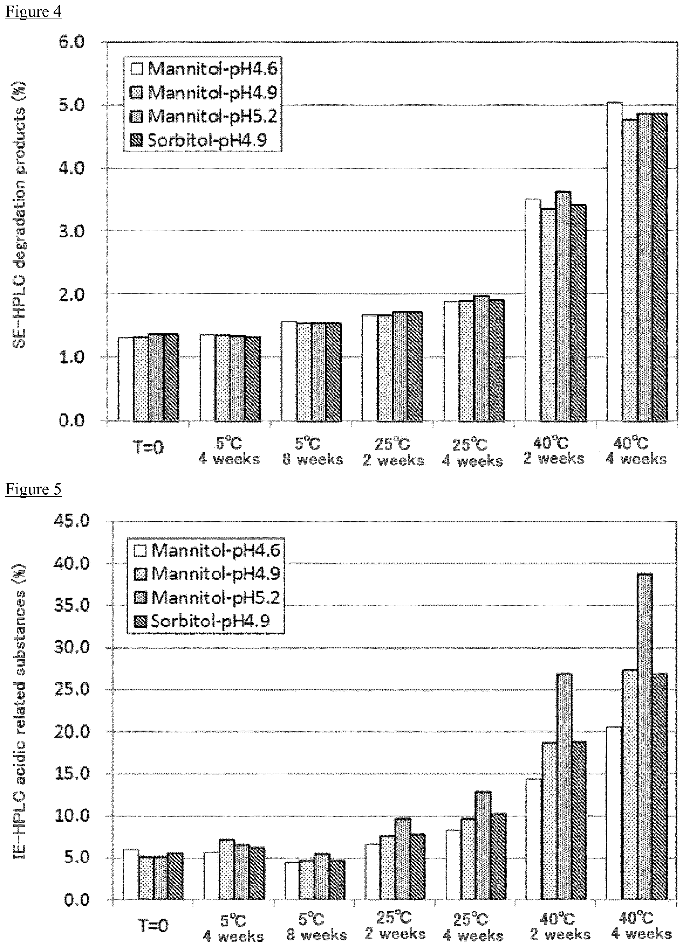

The results are shown in Figure 3 to Figure 5.

It was confirmed that both the D-mannitol formulation and the D-sorbitol formulation were stable within the range of pH 4.6 to 5.2.

### <<Example 6: Stabilization effect by selection of buffers in formulations containing isotonic agent and surfactant>>

With respect to the two formulations (samples No. E-1 and No. E-2) shown in Table 5, the stability after storage under each condition (5°C, 25°C, and 40°C) for 4 weeks in an upright position was evaluated. The samples prepared in accordance with each formulation were sterilely filtered through a 0.22 µm filter, and glass vials (3 mL volume) were filled with 1.3 mL of each filtrate, and capping with rubber stoppers and crimping were carried out.

In connection with this, hydrochloric acid and/or sodium hydroxide were added during buffer preparation as pH adjusters, if necessary, so as to become a desired pH.

The multimers and the degradation products were measured by the SE-HPLC method, and the acidic related substances were measured by the IE-HPLC method. The SE-HPLC method and the IE-HPLC method were carried out in a similar manner to Example 1.

The results are shown in Table 6.

Both E-1 and E-2 were stable.

**[Table 5]**

| Sample No. | Active ingredient | Buffer | pH | Isotonic agent | Surfactant |
|---|---|---|---|---|---|
| E-1 | PEGylated anti-human NGF antibody Fab' fragment 20 mg/mL (converted to Fab') | Acetic acid 20 mmol/L | 4.9 | Sorbitol 240 mmol/L | Polysorbate 80 0.02% (w/v) |
| E-2 | | Citric acid 20 mmol/L | | | |

**[Table 6]**

| Sample No. | Items to be analyzed | Initial | Storage 5°C 4 weeks | Storage 25°C 4 weeks | Storage 40°C 4 weeks |
|---|---|---|---|---|---|
| E-1 | Multimers (%) | 5.00 | 4.93 | 4.87 | 5.13 |
| | Degradation products (%) | 0.14 | 0.15 | 0.67 | 3.78 |
| | Acidic related substances (%) | 1.27 | 3.62 | 8.36 | 28.91 |
| E-2 | Multimers (%) | 5.36 | 5.15 | 5.02 | 5.29 |
| | Degradation products (%) | 0.14 | 0.27 | 0.73 | 3.93 |
| | Acidic related substances (%) | 1.29 | 3.31 | 7.05 | 23.67 |

### <<Example 7: Stability evaluation of formulation containing buffer, isotonic agent, and surfactant>>

With respect to the formulation shown in Table 7 prepared by adding the PEGylated anti-human NGF antibody Fab' fragment (so as to become 40 mg/mL in terms of Fab'-PEG (approximately 20 mg/mL in terms of Fab')), D-sorbate (so as to become 240 mmol/L), and polysorbate 80 (so as to become 0.02% (w/v)), to a formulation liquid at pH 4.9 prepared using 20 mmol/L citric acid, the stability after freeze-thaw stress, shaking stress, or light stress, and the stability after storage under each condition (-20°C, 5°C, and 25°C) for 3 months in an upright position, were evaluated. In connection with this, citric acid and trisodium citrate dehydrate were added so as to become 20 mmol/L in total as citric acid, and at a ratio such that the pH was 4.9. Hydrochloric acid or sodium hydroxide was added during buffer preparation so as to become pH 4.6 to 5.2, if necessary. The sample was sterilely filtered through a 0.22 µm filter, and glass vials (3 mL volume) were filled with 1.2 mL of the filtrate, and capping with rubber stoppers and crimping were carried out. The vials were stored under each condition.

The freeze-thaw test was carried out in a similar manner to Example 3.

The shaking stability test was carried out by placing the samples in a shaker (RECIPRO SHAKER SR-1; manufactured by TAITEC) in a horizontal position, and shaking them at room temperature at a speed of 150 rpm for 48 hours.

The light stability test was carried out by storing the samples in a horizontal position under light exposure at 1000 lux for up to 48 hours with D65 lamp.

The appearance was visually inspected, pH was measured using a pH meter, the multimers and the degradation products were measured by the SE-HPLC method, the acidic related substances were measured by the IE-HPLC method, and the average radius and the polydispersity were measured by the DLS method.

The visual inspection was carried out under the room fluorescent light, pH was measured using a pH meter calibrated using a standard solution for calibration, the SE-HPLC method and the IE-HPLC method were carried out in a similar manner to Example 1, and the DLS method was carried out in a similar manner to Example 2.

The results are shown in Table 8 and Table 9. The formulation was stable at each storage condition.

**[Table 7]**

| Component | Function | Weight (/vial) |
|---|---|---|
| PEGylated anti-human NGF antibody Fab' fragment | Active ingredient | 40 mg (as Fab'-PEG) |
| Citric acid | Buffer | 1.58 mg |
| Trisodium citrate dihydrate | Buffer | 3.47 mg |
| D-Sorbitol | Isotonic agent | 43.72 mg |
| Polysorbate 80 | Surfactant | 0.20 mg |
| Hydrochloric acid | pH adjustor | q.s. to pH 4.9 |
| Sodium hydroxide | pH adjustor | |
| Water for injection | Solvent | q.s. to 1 mL in total |

| | | |
|---|---|---|
| q.s.: quantum sufficit | | |

**[Table 8]**

| Test | | Storage conditions | | | |
|---|---|---|---|---|---|
| | | Initial | Storage -20°C 3 months | Storage 5°C 3 months | Storage 25°C 3 months |
| Appearance | | No foreign insoluble matter appeared | No foreign insoluble matter appeared | No foreign insoluble matter appeared | No foreign insoluble matter appeared |
| pH | | 4.89 | 4.91 | 4.92 | 4.92 |
| SE-HPLC | Multimers (%) | 2.83 | 2.97 | 3.09 | 3.83 |
| | Degradation products (%) | 2.04 | 2.06 | 2.02 | 3.83 |
| IE-HPLC | Acidic related substances (%) | 7.39 | 6.58 | 7.59 | 15.82 |
| DLS | Average radius (nm) | 4.4 | 4.5 | 4.5 | 4.6 |
| | Polydispersity (%) | 16.5 | 16.0 | 15.4 | 18.3 |

**[Table 9]**

| Test | | Storage conditions | | | |
|---|---|---|---|---|---|
| | | Initial | Freeze and thaw | Shaking | Light |
| Appearance | | No foreign insoluble matter appeared | No foreign insoluble matter appeared | No foreign insoluble matter appeared | No foreign insoluble matter appeared |
| pH | | 4.89 | 4.90 | 4.90 | 4.90 |
| SE-HPLC | Multimers (%) | 2.94 | 2.97 | 2.98 | 3.23 |
| | Degradation products (%) | 1.96 | 2.05 | 2.08 | 2.13 |
| IE-HPLC | Acidic related substances (%) | 7.23 | 7.17 | 7.30 | 7.75 |
| DLS | Average radius (nm) | 4.4 | 4.5 | 4.5 | 4.4 |
| | Polydispersity (%) | 16.5 | 15.4 | 16.4 | 16.7 |

### <<Example 8: Stabilization effect by selection of optimum pH in high-concentration formulations>>

In order to adjust the concentration of the PEGylated anti-human NGF antibody Fab' fragment in terms of Fab'-PEG to 100 mg/mL (approximately 50 mg/mL in terms of Fab'), and in order to obtain the formulations shown in Table 10, buffer exchange and concentration were carried out using a spin column, and polysorbate 80 was added so as to be 0.02% (w/v), and each formulation was filtered through a 0.22 µm filter. Glass vials (3 mL volume) were filled with 0.25 mL or 1.15 mL of each filtrate, and capping with rubber stoppers and crimping were carried out, and the storage stability (the amount of multimers, the amount of degradation products, and the amount of acidic related substances) of the PEGylated anti-human NGF antibody Fab' fragment at 25°C or 40°C was evaluated.

In connection with this, sodium hydroxide was added during buffer preparation as pH adjusters, if necessary, so as to become a desired pH.

The multimers and the degradation products were measured by the SE-HPLC method, and the acidic related substances were measured by the icIEF method.

In the SE-HPLC measurement, a TSKgel (registered trademark) G3000 SWXL column (manufactured by Tosoh) was connected to an HPLC system. A mobile phase prepared by adding acetonitrile, so as to be 17.5%, to a solution of 20 mmol/L phosphoric acid/400 mmol/L sodium chloride pH 7.0, was flowed at a flow rate of 0.5 mL/min. Each sample was injected in an amount of 50 µg in terms of Fab'-PEG. The column temperature was set to 30°C, and the sample temperature was set to 5°C. The detection was carried out at UV 280 nm.

In the icIEF measurement, an icIEF Cartridge FC-Coated (manufactured by Protein Simple) was connected to an iCE3 system, and the measurement was carried out. Samples for measurement were prepared by mixing 120 µL of a sample matrix consisting of approximately 0.2% methylcellulose, approximately 2.7% Pharmalyte 3-10, approximately 1.6% Pharmalyte 8-10.5, approximately 1.1% CHAPS, approximately 0.5% pI marker 5.85, and approximately 0.5% pI marker 9.46, with 5 µL of each sample diluted with ultrapure water so that the concentration of the PEGylated anti-human NGF antibody Fab' fragment was 10 mg/mL. Prefocusing was carried out at 1500 V for a minute, and, focusing was carried out at 3000 V for 16 minutes. The detection was carried out at UV 280 nm.

The measurement results of the amount of multimers, the degradation products, and the acidic related substances were shown in Tables 11 to 13, respectively. Even in the high concentration formulations, there was a tendency that the amounts of the multimers, the degradation products, and the acidic related substances increased as the pH was higher.

**[Table 10]**

| Sample No. | Buffer | pH | Surfactant |
|---|---|---|---|
| F-1 | 10 mmol/L Citric acid | 4.6 | 0.02% (w/v) Polysorbate 80 |
| F-2 | | 4.9 | |
| F-3 | | 5.2 | |
| F-4 | | 5.5 | |
| F-5 | | 5.8 | |
| F-6 | | 6.0 | |
| F-7 | | 6.3 | |
| F-8 | 10 mmol/L Phosphoric acid | 6.0 | |
| F-9 | 10 mmol/L Acetic acid | 4.9 | |
| F-10 | 10 mmol/L Histidine | 6.0 | |

**[Table 11]**

| Sample No. | Buffer | pH | Initial | Storage 25°C 2 weeks | Storage 25°C 4 weeks | Storage 40°C 2 weeks | Storage 40°C 4 weeks |
|---|---|---|---|---|---|---|---|
| F-1 | Citric acid | 4.6 | 2.1 | 2.4 | 2.9 | 2.9 | 3.6 |
| F-2 | | 4.9 | 2.2 | 2.6 | 3.1 | 3.2 | 3.7 |
| F-3 | | 5.2 | 2.3 | 2.8 | 3.2 | 3.4 | 3.8 |
| F-4 | | 5.5 | 2.5 | 3.1 | 3.5 | 3.7 | 4.2 |
| F-5 | | 5.8 | 2.6 | 3.3 | 3.7 | 3.9 | 4.4 |
| F-6 | | 6.0 | 2.7 | 3.5 | 3.9 | 4.2 | 4.6 |
| F-7 | | 6.3 | 3.0 | 3.8 | 4.2 | 4.6 | 5.0 |
| F-8 | Phosphoric acid | 6.0 | 2.6 | 3.2 | 3.5 | 3.7 | 4.2 |
| F-9 | Acetic acid | 4.9 | 2.2 | 2.7 | 2.9 | 3.3 | 3.7 |
| F-10 | Histidine | 6.0 | 2.5 | 3.1 | 3.4 | 3.6 | 4.0 |

| | | | | | | | |
|---|---|---|---|---|---|---|---|
| Unit: % | | | | | | | |

**[Table 12]**

| Sample No. | Buffer | pH | Initial | Storage 25°C 2 weeks | Storage 25°C 4 weeks | Storage 40°C 2 weeks | Storage 40°C 4 weeks |
|---|---|---|---|---|---|---|---|
| F-1 | Citric acid | 4.6 | 0.4 | 0.8 | 1.2 | 2.3 | 3.8 |
| F-2 | | 4.9 | 0.5 | 0.9 | 1.2 | 2.3 | 3.6 |
| F-3 | | 5.2 | 0.5 | 0.9 | 1.2 | 2.4 | 3.7 |
| F-4 | | 5.5 | 0.5 | 1.0 | 1.3 | 2.6 | 4.0 |
| F-5 | | 5.8 | 0.6 | 1.1 | 1.5 | 2.9 | 4.3 |
| F-6 | | 6.0 | 0.6 | 1.3 | 1.6 | 3.3 | 4.5 |
| F-7 | | 6.3 | 0.7 | 1.4 | 1.8 | 3.6 | 4.9 |
| F-8 | Phosphoric acid | 6.0 | 0.6 | 1.1 | 1.4 | 2.7 | 3.9 |
| F-9 | Acetic acid | 4.9 | 0.6 | 0.9 | 1.2 | 2.2 | 3.4 |
| F-10 | Histidine | 6.0 | 0.6 | 1.3 | 1.7 | 3.1 | 4.5 |

| | | | | | | | |
|---|---|---|---|---|---|---|---|
| Unit: % | | | | | | | |

**[Table 13]**

| Sample No. | Buffer | pH | Initial | Storage 25°C 2 weeks | Storage 25°C 4 weeks | Storage 40°C 2 weeks | Storage 40°C 4 weeks |
|---|---|---|---|---|---|---|---|
| F-1 | Citric acid | 4.6 | 12.6 | 19.7 | 16.2 | 19.9 | 24.4 |
| F-2 | | 4.9 | 13.9 | 18.1 | 18.4 | 22.8 | 28.7 |
| F-3 | | 5.2 | 12.9 | 20.1 | 20.1 | 27.1 | 35.4 |
| F-4 | | 5.5 | 12.8 | 22.8 | 24.0 | 34.4 | 44.4 |
| F-5 | | 5.8 | 14.3 | 26.0 | 31.2 | 47.3 | 54.4 |
| F-6 | | 6.0 | 13.6 | 29.0 | 37.8 | 55.8 | 60.5 |
| F-7 | | 6.3 | 14.1 | 35.8 | 47.4 | 66.1 | 65.8 |
| F-8 | Phosphoric acid | 6.0 | 13.3 | 26.0 | 30.9 | 41.1 | 49.0 |
| F-9 | Acetic acid | 4.9 | 13.4 | 19.4 | 22.2 | 25.7 | 35.0 |
| F-10 | Histidine | 6.0 | 14.9 | 34.4 | 44.4 | 54.3 | 59.1 |

| | | | | | | | |
|---|---|---|---|---|---|---|---|
| Unit: % | | | | | | | |

### <<Example 9: Study of stability evaluation of high-concentration formulations containing buffer, isotonic agent, and surfactant>>

In a similar manner to Example 8, with respect to the formulations shown in Table 14 prepared by adding the PEGylated anti-human NGF antibody Fab' fragment (so as to become 100 mg/mL in terms of Fab'-PEG (approximately 50 mg/mL in terms of Fab')), D-sorbate (so as to become 240 mmol/L), L-arginine hydrochloride (so as to become 140 mmol/L), and polysorbate 80 (so as to become 0.02% (w/v)), to formulation liquids shown in Table 14 prepared using 20 mmol/L citric acid, the stability after storage under each condition (5°C and 25°C) for 1 month or 3 months in an upright position were evaluated. The prepared samples were sterilely filtered through a 0.22 µm filter, and glass vials (3 mL volume) were filled with 0.8 mL of the filtrate, and capping with rubber stoppers and crimping were carried out. The vials were stored under each condition.

The multimers and the degradation products were measured by the SE-HPLC method, and the acidic related substances and the basic related substances were measured by the icIEF method. The SE-HPLC method and the icIEF method were carried out in a similar manner to Example 8.

The results are shown in Table 15. The high-concentration formulation containing citric acid, arginine, and polysorbate, or the high-concentration formulation containing citric acid, D-sorbitol, and polysorbate were stable, even after storage.

**[Table 14]**

| Component | Function | G-1 | G-2 |
|---|---|---|---|
| | | Weight (/vial) | Weight (/vial) |
| PEGylated anti-human NGF antibody Fab' fragment | Active ingredient | 100 mg | 100 mg |
| Citric acid | Buffer | 1.58 mg | 1.58 mg |
| Trisodium citrate dihydrate | Buffer | 3.47 mg | 3.47 mg |
| L-Arginine hydrochloride | Isotonic agent | 29.50 mg | - |
| D-Sorbitol | Isotonic agent | - | 43.72 mg |
| Polysorbate 80 | Surfactant | 0.20 mg | 0.20 mg |
| Hydrochloric acid | pH adjustor | q.s. to pH 5.0 | q.s. to pH 4.9 |
| Sodium hydroxide | pH adjustor | | |
| Water for injection | Solvent | q.s. to 1 mL in total | q.s. to 1 mL in total |

| | | | |
|---|---|---|---|
| q.s.: quantum sufficit | | | |

**[Table 15]**

| Sample No. | | Initial | Storage 5°C 1 month | Storage 5°C 3 months | Storage 25°C 1 month | Storage 25°C 3 months |
|---|---|---|---|---|---|---|
| G-1 | Multimers (%) | 2.9 | 2.5 | 2.6 | 2.8 | 3.1 |
| | Degradation products (%) | 0.7 | 0.5 | 0.6 | 1.0 | 1.8 |
| | Acidic related substances (%) | 15.7 | 14.6 | 15.1 | 19.4 | 23.6 |
| | Basic related substances (%) | 5.2 | 5.6 | 6.6 | 8.5 | 15.0 |
| G-2 | Multimers (%) | 2.9 | 2.8 | 2.9 | 3.1 | 3.5 |
| | Degradation products (%) | 0.6 | 0.5 | 0.6 | 0.9 | 1.8 |
| | Acidic related substances (%) | 15.9 | 14.3 | 14.7 | 17.9 | 23.5 |
| | Basic related substances (%) | 5.3 | 5.7 | 7.1 | 9.1 | 14.4 |

### INDUSTRIAL APPLICABILITY

According to the present invention, a stable pharmaceutical composition comprising the PEGylated anti-human NGF antibody Fab' fragment, more particularly, a stable pharmaceutical composition comprising the PEGylated anti-human NGF antibody Fab' fragment, capable of inhibiting the generation of its degradation products, multimers, acidic related substances, and basic related substances, can be provided.

Although the present invention has been described with reference to specific embodiments, various changes and modifications obvious to those skilled in the art are possible without departing from the scope of the appended claims.

### FREE TEXT IN SEQUENCE LISTING

The sequence of SEQ ID NO: 1 is the amino acid sequence of a heavy chain fragment of an anti-human NGF antibody Fab' fragment, the sequence of SEQ ID NO: 2 is the nucleotide sequence of a heavy chain fragment gene of the anti-human NGF antibody Fab' fragment, the sequence of SEQ ID NO: 3 is the amino acid sequence of the light chain of the anti-human NGF antibody Fab' fragment, and the sequence of SEQ ID NO: 4 is the nucleotide sequence of the light chain gene of the anti-human NGF antibody Fab' fragment.

## Claims

1. A stable pharmaceutical composition comprising a PEGylated anti-human NGF antibody Fab' fragment,
wherein pH is 4 to 5.5;
wherein the anti-human NGF antibody Fab' fragment is selected from:
(1) an anti-human NGF antibody Fab' fragment comprising a heavy chain fragment consisting of the amino acid sequence of SEQ ID NO: 1, and a light chain consisting of the amino acid sequence of SEQ ID NO: 3, and
(2) an anti-human NGF antibody Fab' fragment that is a Fab' fragment generated by a post-translational modification of the anti-human NGF antibody Fab' fragment of (1); and wherein the PEGylation is a covalent bond of a thiol reactive group of a PEG derivative, to which the thiol reactive group is bonded, to a thiol group of cysteine in a hinge region of the anti-human NGF antibody Fab' fragment.

2. The pharmaceutical composition according to claim 1, further comprising a pharmaceutically acceptable buffer, a pharmaceutically acceptable isotonic agent, and a pharmaceutically acceptable surfactant.

3. The pharmaceutical composition according to claim 1 or 2, wherein pH is 4.5 to 5.5.

4. The pharmaceutical composition according to any one of claims 1 to 3, wherein the pharmaceutically acceptable buffer is one, or two or more selected from the group consisting of citric acid, acetic acid, and histidine, and pharmaceutically acceptable salts thereof.

5. The pharmaceutical composition according to any one of claims 1 to 4, wherein the pharmaceutically acceptable buffer is citric acid, or a pharmaceutically acceptable salt thereof.

6. The pharmaceutical composition according to any one of claims 1 to 5, wherein a concentration of the pharmaceutically acceptable buffer is 1 to 200 mmol/L.

7. The pharmaceutical composition according to any one of claims 1 to 6, wherein the pharmaceutical composition is a liquid preparation or a lyophilized preparation.

8. The pharmaceutical composition according to any one of claims 1 to 7, wherein the pharmaceutical composition is a liquid preparation.

9. The pharmaceutical composition according to any one of claims 1 to 8, wherein the pharmaceutically acceptable isotonic agent is one, or two or more selected from the group consisting of arginine, D-sorbitol, D-mannitol, trehalose, sucrose, xylitol, erythritol, threitol, inositol, dulcitol, arabitol, isomalt, lactitol, and maltitol.

10. The pharmaceutical composition according to any one of claims 1 to 9, wherein a concentration of the pharmaceutically acceptable isotonic agent is 1 to 500 mmol/L.

11. The pharmaceutical composition according to any one of claims 1 to 10, wherein the pharmaceutically acceptable surfactant is at least one of polysorbate or poloxamer.

12. The pharmaceutical composition according to any one of claims 1 to 11, wherein the pharmaceutically acceptable surfactant is polysorbate 80.

13. The pharmaceutical composition according to any one of claims 1 to 12, wherein a concentration of the pharmaceutically acceptable surfactant is 0.001 to 1% (w/v).

14. The pharmaceutical composition according to any one of claims 1 to 13, wherein a concentration of the PEGylated anti-human NGF antibody Fab' fragment is 0.1 to 200 mg/mL.

15. The pharmaceutical composition according to claim 1, comprising, as the PEGylated anti-human NGF antibody Fab' fragment, an anti-human NGF antibody Fab' fragment PEGylated by covalently bonding a thiol reactive group of a PEG derivative, to which the thiol reactive group is bonded, to a thiol group of cysteine in a hinge region of an anti-human NGF antibody Fab' fragment comprising a heavy chain fragment consisting of the amino acid sequence of SEQ ID NO: 1, and a light chain consisting of the amino acid sequence of SEQ ID NO: 3.

16. The pharmaceutical composition according to claim 1, comprising, as the PEGylated anti-human NGF antibody Fab' fragment, an anti-human NGF antibody Fab' fragment PEGylated by covalently bonding a thiol reactive group of a PEG derivative, to which the thiol reactive group is bonded, to a thiol group of cysteine in a hinge region of an anti-human NGF antibody Fab' fragment that is a Fab' fragment generated by a post-translational modification of an anti-human NGF antibody Fab' fragment comprising a heavy chain fragment consisting of the amino acid sequence of SEQ ID NO: 1, and a light chain consisting of the amino acid sequence of SEQ ID NO: 3.

17. The pharmaceutical composition according to claim 1 or 16, wherein the post-translational modification of an anti-human NGF antibody Fab' fragment is pyroglutamylation at the N-terminus of a heavy chain variable region.

18. A method of stabilizing a PEGylated anti-human NGF antibody Fab' fragment, **characterizing by** adjusting a pH to 4 to 5.5,
wherein the anti-human NGF antibody Fab' fragment is selected from:
(1) an anti-human NGF antibody Fab' fragment comprising a heavy chain fragment consisting of the amino acid sequence of SEQ ID NO: 1, and a light chain consisting of the amino acid sequence of SEQ ID NO: 3, and
(2) an anti-human NGF antibody Fab' fragment that is a Fab' fragment generated by a post-translational modification of the anti-human NGF antibody Fab' fragment of (1); and wherein the PEGylation is a covalent bond of a thiol reactive group of a PEG derivative, to which the thiol reactive group is bonded, to a thiol group of cysteine in a hinge region of the anti-human NGF antibody Fab' fragment.

19. The method according to claim 18, using a pharmaceutically acceptable buffer, a pharmaceutically acceptable isotonic agent, and a pharmaceutically acceptable surfactant.
